# EUROPEAN PATENT APPLICATION

(11) **EP 3 241 895 A1**
(43) Date of publication of application: **08.11.2017**
(21) Application number: 16191245.6
(22) Date of filing: 28.09.2016
(51) Int. Cl.: C12N 5/00, C12N 5/079, C12N 5/0793, C12N 5/0797, C12N 5/077, C12N 5/0775

(54) **TRANSGLUTAMINASE MEDIATED HIGH MOLECULAR WEIGHT HYALURONAN HYDROGELS**

(30) Priority: 04.05.2016 EP 16168457
(71) Applicant: ETH Zürich, ETH Transfer, 8092 Zürich (CH)
(72) Inventor: BROGUIERE, Nicolas, 8004 Zürich (CH); CAVALLI, Emma, 8050 Zürich (CH); ZENOBI-WONG, Marcy, 8049 Zurich (CH)
(74) Representative: Schulz Junghans Patentanwälte PartGmbB

(57) **Abstract**

The invention relates to a process for forming a hyaluronan hydrogel, comprising the steps of providing a hyaluronan donor peptide conjugate and a hyaluronan acceptor peptide conjugate each represented by a general formula I, wherein 10% of R¹ moieties are represented by a general formula II, wherein L is a 2 to 6 atom linker moiety and Pep is a transglutaminase donor or acceptor peptide, and the rest of R¹ moieties are represented by-COOH, and then adding a factor XIII polypeptide and a thrombin polypeptide, or a factor XIIIa polypeptide.

The invention further relates to compositions and hydrogels characterized by the depicted chemistry.

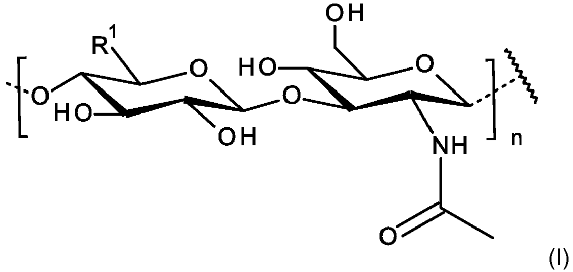

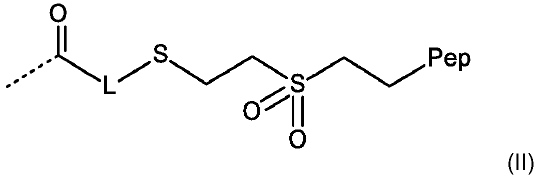

## Description

Hyaluronan (HA) is an abundant extracellular matrix component of connective, epithelia and central nervous system tissues. In cartilage HA together with aggrecan and link protein form large, negatively-charged aggregating structures important for the mechanical properties of cartilage. In the central nervous system, HA is the backbone of the brain and spinal cord extracellular matrix (ECM). Furthermore, the ECM serves as a template for morphogen and growth factor presentation, and variations in its composition are recognized by specialized cell receptors, thereby providing guidance during neural development, plasticity, and regeneration. HA in particular is known to regulate inflammation, with low molecular weight (MW) fragments being pro-inflammatory and high MW chains being anti-inflammatory. High MW HA even limits the glial scarring after brain damage and spinal cord injury (SCI). It is also an important component for maintaining neural stem cells *in vitro* and *in vivo.*

HA has become a popular tool for cartilage and neural tissue engineering. Standard methods to form HA gels are thiolated HA (HA-SH) cross-linked with acrylated PEG by Michael addition, methacrylated HA (HA-MA) photo-crosslinked with a radical initiator, and adipic dihydrazide (ADH) cross-linked HA (HA-ADH). These cross-linking schemes have major drawbacks which have limited their application. HA gels formed by Michael addition are injectable systems. At physiological pH they gel slowly, making their handling difficult and their clinical translation unlikely. For example, investigators needed 25 min of pre-reaction before injecting such gels into the brain (Liang et al. Biomaterials, vol. 34, no. 22, pp. 5521-5529, 2013). At high pH, where the reaction proceeds quickly, cell viability is affected. Thiolated HA also readily oxidizes to disulfides, with significant loss in a matter of hours when in solution at physiological pH. This becomes particularly problematic with high MW HA-SH (more than 1 MDa), as it tends to get oxidized to the extent that it forms an insoluble sponge, even when stored frozen or in dry form. This has prompted most users of HA-SH to work with low MW, typically around 100 kDa (Zheng Shu et al. Biomaterials, vol. 25, no. 7-8, pp. 1339-1348, Mar. 2004). Conversely, photo-cross-linking of HA-MA with a radical initiator has the advantage of being based on reagents with higher stability and very fast gelation, but the need for light exposure means such gels are not injectable. Additionally, free radicals are very detrimental to cell viability. It was for example found that neural progenitor cells survive no more than 90 s during UV exposure in the presence of Irgacure 2959 (Seidlits et al. Biomaterials, vol. 31, no. 14, pp. 3930-40, May 2010), which prompted the addition of a precross-linking step before mixing in the cells for encapsulation. Finally, ADH cross-linking of HA can only be done in harsh conditions not compatible with neuron encapsulation or in situ gelling (pH 3.5 - 5 and carboxylic acid activation with a carbodiimide). This makes this method incompatible with in situ gelation and/or cell encapsulation.

Another FXIIIa cross-linkable HA derivative was recently introduced by Ranga et al (Biomacromolecules 2016, 10.1021/acs.biomac.5b01587), which is based on low MW HA co-cross-linked with polyethylene glycol (PEG). This gel is characterized by lack of stability at concentrations below 1% (w/v).

The objective of the present invention is to overcome the mentioned deficiencies in the state of the art. This objective is attained by the subject matter of the independent claims.

The present invention provides for the first time high molecular weight hyaluronan gel (HA-TG) gel which crosslinks using a specific transglutaminase (TG) activity, particularly that of the activated blood coagulation factor XIII (FXIIIa). This HA-TG gel is injectable and has a gelling speed tunable depending on the amount of enzyme added. Gel formation kinetics can be brought down to <40s, which makes it very attractive for demanding applications where bleeding and movement may occur. Due to the specific recognition of FXIIIa substrate peptides by the enzyme, the cross-linking is completely free of toxic chemicals. FXIIIa is also capable of covalently cross-linking fibrin, its native function, which gives the possibility to co-cross-link such HA derivatives with fibrin(ogen), and also ensures good tissue adhesion. Furthermore, the modified HA is chemically inert, as it is modified with peptides that are not susceptible to oxidation, hydrolysis, or intramolecular Michael addition, unlike thiol or (meth)acrylate derivatives. This guarantees very high stability (no change is visible on the rheological measurements after several months in solution at physiological pH or after more than one year in frozen dry form). Finally, it is possible to work directly with high MW HA (1-2 MDa) without risk of spontaneous cross-linking in storage. This cross-linking method was used recently to cross-link PEG gels (Ehrbar et al., Biomaterials, vol. 28, no. 26, pp. 3856-66, Sep. 2007) used in bone tissue engineering applications. Enzymatically cross-linked hydrogels have been extensively applied for drug delivery and cell encapsulation, though the most widespread enzymatic systems have lower bioorthogonality (e.g. HRP-tyramine requires hydrogen peroxide, lysyl oxidase creates aldehydes, and most transglutaminase work uses less specific and more immunogenic bacterial transglutaminases) (Teixeira et al., Biomaterials, vol. 33, no. 5, pp. 1281-90, Mar. 2012).

### Terms and definitions

Hyaluronan (also referred to as hyaluronate or hyaluronic acid) is a glycosaminoglycan naturally occurring in the human body. Its structure is that of a polymer of a repeat unit comprising a D-glucuronic acid moiety and a N-acetylglucosamine moiety in alternating ß-1,3 and ß-1,4 linkage. The general formula is

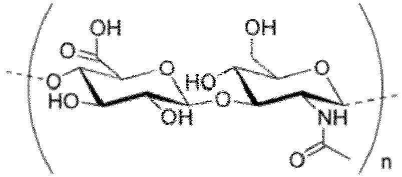

The molecular weight of the repeat unit is 379 g/mol. Hyaluronan in the body can exceed 2500 repeat units (n) per molecule.

The term Factor XIII polypeptide (Laki Lorand factor, fibrin stabilizing factor) in the context of the present specification relates to factor XIII, a transglutaminase occurring in humans, which is a heterotetramer of two catalytic A subunits (UniProt No. P00488) and two carrier B subunits (UniProt No. P05160), or to a functional equivalent thereof.

The term thrombin polypeptide in the context of the present specification relates to a factor (*fibrinogenase, thrombase, activated blood-coagulation factor II, blood-coagulation factor IIa, factor IIa*) that activates factor XIII in the human coagulation cascade (UniProt No. P00734), or a functional equivalent thereof.

The term transglutaminase in the context of the present specification relates to an enzyme that catalyses the formation of an isopeptide bond between a free amine group on the side chain of a lysine residue comprised in a transglutaminase acceptor peptide and the acyl group on the side chain of a glutamine residue comprised in a transglutaminase donor peptide. Examples of human transglutaminases are keratinocyte transglutaminase (Uniprot P22735), tissue transglutaminase (UniProt P21980), epidermal transglutaminase and prostate transglutaminase.

The term transglutaminase donor peptide in the context of the present specification relates to a peptide sequence efficiently reacting with a transglutaminase acceptor peptide in presence of a particular transglutaminase enzyme. Donor and acceptor need to be selected for the particular transglutaminase. For many transglutaminases, donor and acceptor sequences are known. Methods for determining transglutaminase substrate (donor and acceptor) sequences are known in the art (Keresztessy et al., Protein Science 2006, Vol 15(11), 2466-2480).

The term chondoprogenitor cell in the context of the present specification relates to a stem cell partially differentiated towards cartilage lineage.

The term chondrocyte in the context of the present specification relates to a mature cartilage cell, particularly a mature cartilage cell from articular, auricular, nasal, costal, meniscus, nucleous pulposus, disc, used from autologous or allogeneic source. Chondroprogenitor cells from these tissues and perichondrium similarly can be referred to as chondroprogenitor cells, as can be stem cells from bone marrow, cartilage, fat, and blood.

The term chondrogenic cell in the context of the present specification is used as a generic term that encompasses chondrocytes and chondroprogenitor cells and specifically relates to cells able to produce cartilage and collagen type II and aggrecan.

Amino acid sequences are given from amino to carboxyl terminus. Capital letters for sequence positions refer to L-amino acids in the one-letter code (Stryer, Biochemistry, 3rd ed. p.21).

A C₁-C₄ alkyl in the context of the present invention signifies a saturated linear or branched hydrocarbon having 1, 2, 3 or 4 carbon atoms, wherein one carbon-carbon bond may be unsaturated and one CH₂ moiety may be exchanged for oxygen (ether bridge). Non-limiting examples for a C₁-C₄ alkyl are methyl, methylene, ethyl, ethylene, propyl, propylene, prop-2-enyl, n-butyl, 2-methylpropyl, *tert*-butyl, but-3-enyl, prop-2-inyl and but-3-inyl.

A C₁-C₅ alkyl in the context of the present invention signifies a saturated linear or branched hydrocarbon having 1, 2, 3, 4 or 5 carbon atoms, wherein one carbon-carbon bond may be unsaturated and one CH₂ moiety may be exchanged for oxygen (ether bridge). Non-limiting examples for a C₁-C₅ alkyl include the examples given for C₁-C₄ alkyl above, and additionally 3-methylbut-2-enyl, 2-methylbut-3-enyl, 3-methylbut-3-enyl, n-pentyl, 2-methylbutyl, 3-methylbutyl, 1,1-dimethylpropyl, 1,2-dimethylpropyl, 1,2-dimethylpropyl and pent-4-inyl.

According to a first aspect, the invention provides a process for forming a hyaluronan hydrogel. This process comprises the steps of
a. Providing an aqueous solution of a first hyaluronan peptide conjugate, which comprises transglutaminase (TG) donor peptides and a second hyaluronan peptide conjugate, which comprises transglutaminase acceptor peptides. TG donor and TG acceptor peptides are present in approximately equimolar amounts. If a factor XIII derived transglutaminase is to be used in the subsequent covalent linking step, the donor and acceptor peptides need to be suitable substrates of factor XIIIa.
   The first hyaluronan peptide conjugate and said second hyaluronan peptide conjugate are each represented by a general formula I, 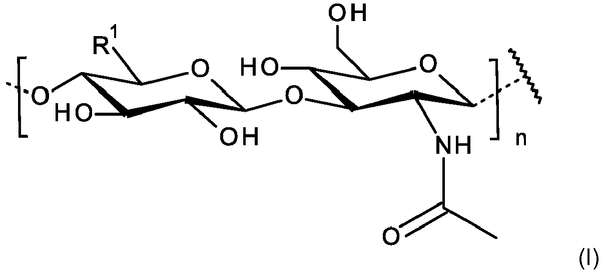 wherein 5% to 20%, particularly 8-12%, more particularly approximately 10% of R¹ moieties are represented by a general formula II, 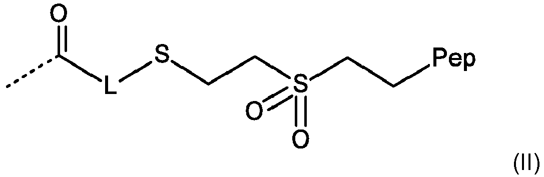 wherein
   - L is a linker moiety having a molecular mass of ≤150g/mol, particularly a linker consisting of 2, 3, 4, 5 or 6 C, N and/or O atoms in the linking chain (plus hydrogen atoms H as applicable to saturate the structure), more particularly L is NH-Alk or O-Alk or NH-NH-CO-Alk with Alk being a C₁, C₂, C₃, C₄ or C₅ alkyl, even more particularly L is -N-NHCO-(CH₂)₂-, and
   - Pep is a transglutaminase donor peptide or a transglutaminase acceptor peptide, respectively, and the rest of R¹ moieties are represented by -COOH.
b. Adding a transglutaminase capable of covalently linking said transglutaminase donor peptides to transglutaminase acceptor peptides, particularly
   i. a factor XIII polypeptide and a thrombin polypeptide, or
   ii. a factor XIIIa polypeptide
to said aqueous solution.

In certain embodiments, R¹ is:

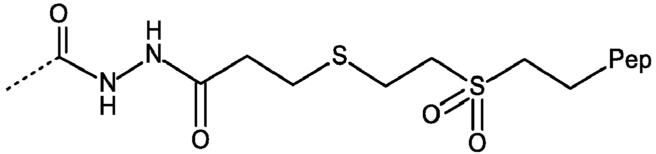

In certain embodiments, the process of the invention provides for heparin-modified (or heparan-sulfate-modified) hyaluronan hydrogels. The resulting gels are of particular utility for one-step surgical treatment of cartilage lesions. For such uses, it is advantageous to include a chondrogenic growth factor (particularly selected from transforming growth factor beta 1, 2, or 3, insulin like growth factor 1 and fibroblast growth factors 1, 9, or 18) to support proliferation and matrix deposition by the cells. Such growth factors may be comprised within the hyaluronan hydrogels. The inventors observed a burst release of growth factor TGF-b1 from hyaluronan gels that do not comprise heparin or heparan sulfate, indicating that pure HA gels do not retain growth factors, thus offering an unfavorable release kinetic for applications that require growth factor incorporation.

In these embodiments, the aqueous solution of step a. additionally comprises heparin or heparan sulfate, particularly at a concentration from 0,05% to 0,5% (w/v relative to the gel). In other particular embodiments, the amount of heparin or heparan sulfate is 2,5% to 15%, more particularly 5%-10% (m/m) in relation to the amount of hyaluronan comprised in the gel.

Both the addition of heparin and the addition of heparan sulfate will work for the uses discussed herein. The inventors employed heparin sodium salt from porcine intestinal mucosa (which is also the source for clinical grade heparin products). As it is unfractionated, the molecular weight of the chains is heterogeneous. For clinical applications, a GMP grade heparin is preferred, particularly the low molecular weight product.

In certain embodiments, the heparin or heparan sulfate comprises covalently attached transglutaminase donor and / or acceptor peptides. The chemistry employed for attaching the peptides can be essentially the same as the process described herein in detail for modifying hyaluronan. In certain embodiments, 10% to 20%, particularly ca. 15%, of carboxylic acid groups present in the heparin or heparan sulfate are covalently modified. In certain more particular embodiments, the carboxylic acid moieties are covalently modified to contain a modification described by general formula (II) as laid out above [wherein the carbon shown on the left is the carboxylic carbon comprised within the glucosaminoglycan backbone], with L, S and Pep having the meaning indicated above.

In certain embodiments, the molecular mass of said heparin or heparan sulfate ranges from 15.000 g/mol to 50.000 g/mol.

Heparin is a clinically used polysaccharide, and its growth factor affinity binding properties, notably in the case of TGF-b1, make adding heparin to the gel an interesting feature. The inventors found that the covalent addition of heparin to the HA gels shown in here, allows for a slower and more sustained release of TGF-b1, subsequently improving the chondrogenesis in HA-TG gels.

In certain embodiments, 1 to 50 U/ml of the factor XIII polypeptide (FXIII) are employed, particularly 5 to 40 U/ml, more particularly 15 to 25 U/ml of FXIII. In certain embodiments, 0.1 to 100 U/ml of the thrombin polypeptide, particularly 1 to 20 U/ml, more particularly 12.5 U/ml are employed.

In certain embodiments, pre-activated FXIIIa is employed at an activity of 1 to 50 U/ml, particularly 5 to 20 U/ml. 5 to 20 U/ml are the enzyme concentrations that result in a gelling speed (of the order of 1 min) of particular use in surgical applications, depending on the amount of polymer used (less HA requires more enzyme to arrive at the same gelling speed). According to an alternative of this first aspect the invention provides a process for forming a hyaluronan hydrogel characterized by second-order gelling kinetics. This process comprises the steps of:
a. providing an aqueous solution of a first hyaluronan peptide conjugate comprising transglutaminase donor peptides and a second hyaluronan peptide conjugate comprising transglutaminase acceptor peptides (in approximately equimolar amounts of donor and acceptor peptides, respectively),
b. adding a thrombin polypeptide to said aqueous solution and allowing equilibration of the resulting mixture;
c. subsequently, adding an unactivated factor XIII polypeptide.

Calcium is an essential cofactor for the enzymes. Ideally, the process is performed at a concentration of 50 mM calcium, which acts as a cofactor for both FXIIIa and thrombin. The useful range of calcium concentration is at least 1 to 100 mM, and may be extended to 0.1 to 300 mM with some impact on reaction rate. The pH is also important: the inventors used a pH of 7.6, but the reaction is expected to work at pH values from 6 to 9.

In certain embodiments, the hyaluronan peptide conjugate mix (acceptor and donor peptide conjugate) is equilibrated with FXIII polypeptide and thrombin is added. In certain embodiments, the hyaluronan peptide conjugate mix (acceptor and donor peptide conjugate) is equilibrated with thrombin and FXIII polypeptide is added. In certain embodiments, calcium is present from the outset; in certain other embodiments, the reaction is started by adding calcium.

The inventors have found that equilibrating the hyaluronan peptide conjugate mix with thrombin and then adding unactivated FXIII gives ideal kinetics, particularly for the surgical applications contemplated herein: ∼2min onset and -10-15 min to reach the maximum stiffness.

In certain embodiments, the process according to this first aspect of the invention is conducted at a concentration of
a. 0.1 to 100 U/ml, particularly 1 to 20 U/ml, more particularly 12.5 U/ml thrombin polypeptide; and
b. 1 to 50 U/ml, particularly 5 to 40 U/ml, more particularly 15 to 25 U/ml of factor XIII polypeptide.

In certain embodiments, the process according to this first aspect of the invention is conducted at a concentration of peptide conjugate, referring thereby to the sum of the first hyaluronan peptide conjugate and the second hyaluronan peptide conjugate with respect to total aqueous gel volume, of 0,25% (w/v) to 5% (w/v), particularly 0,75% to 0,95% or from 0, 5% to 3%, 0,5% to 2%, or 0,5% to 1,5%.

The skilled person will understand that many other couples of transglutaminase precursor and activating factor exist in nature, which might be favourably adapted to practice the present invention at least with respect to some of the potential applications of the gels provided herein. Human tissue transglutaminase in its oxidized form and thioredoxin would constitute another example of such a pair. Bacterial transglutaminases are widely used in the food industry. A non-limiting examples is the combination of TG from Streptomyces mobaraensis (EC 2.3.2.13; SwissProt entry name TGL_STRSS, accession number P81453) and the endoprotease TAMEP, (transglutaminase activating metalloprotease, SwissProt entry name TAMP_STRMB, accession number P83543).

Factor XIII however appears to provide the by far the most interesting transglutaminase for clinical tissue engineering applications, because it is the only human TG already available at pharmaceutical grade in large amounts.

A second aspect of the invention relates to a process for modification of a hyaluronan polymer. The hyaluronan polymer is composed of n dimers of D-glucuronic acid moieties and D-N-acetylglucosamine moieties. The D-glucuronic acid moieties bear reactive carboxylic acid moieties. The process comprises the steps of:
a. thiolation of 5% to 20%, particularly 8-12%, more particularly approximately 10% of said reactive carboxylic acid moieties to yield partially thiolated hyaluronan;
b. reacting said partially thiolated hyaluronan with divinylsulfone to yield vinylsulfone-hyaluronan;
c. reacting said vinylsulfone-hyaluronan with a peptide comprising a cysteine moiety, wherein said peptide comprises a sequence selected from a transglutaminase donor peptide sequence and a transglutaminase acceptor peptide sequence.

In certain embodiments of this process according to the second aspect of the invention, thiolation is effected by:
a. reacting said hyaluronan polymer with 3,3'-dithiobis(propanoic dihydrazide), particularly in the presence of an alkylcarbodiimide at pH 4 to 5.5; followed by
b. reacting the product of step a particularly without further workup with a reducing agent selected from TCEP (tris-2-(carboxyethyl)phosphine), DTT (dithiothreitol) and beta-mercaptoethanol, to yield the partially thiolated hyaluronan.

An alternative of this aspect of the invention relates to a process for modification of a heparin or heparan sulfate polymer. Heparin and heparan sulfate comprise D-glucuronic acid moieties and L-iduronic acid moieties, both of which bear reactive carboxylic acid moieties. These carboxylic acid moieties can be modified by the same process as outlined in the previous paragraphs:
a. thiolation of 5% to 20%, particularly 8-12%, more particularly approximately 10% of said reactive carboxylic acid moieties to yield partially thiolated heparin or heparan sulfate;
b. reacting said partially thiolated hyaluronan with divinylsulfone to yield vinylsulfone-heparin or -heparan sulfate;
c. reacting said vinylsulfone-heparin or vinylsulfone -heparan sulfate with a peptide comprising a cysteine moiety, wherein said peptide comprises a sequence selected from a transglutaminase donor peptide sequence and a transglutaminase acceptor peptide sequence.

In certain embodiments, thiolation is effected by:
a. reacting the heparin or heparan sulfate polymer with 3,3'-dithiobis(propanoic dihydrazide), particularly in the presence of an alkylcarbodiimide; followed by
b. reacting the product of step a. (particularly without further workup) with a reducing agent selected from TCEP (tris-2-(carboxyethyl)phosphine), DTT (dithiothreitol) and beta-mercaptoethanol, to yield the partially thiolated heparin or heparan sulfate.

In certain embodiments of any process provided herein (1^{st} and 2^{nd} aspect) the hyaluronan and/or first hyaluronan peptide conjugate and/or second hyaluronan peptide conjugate are characterized by a mean molecular weight equal or greater than (≥) 250 kg/mol. In certain aspects, this molecular weight exceeds 500 kg/mol, or even 1000 kg/mol (1 MDa). Certain aspects make use of hyaluronan or hyaluronan peptide conjugate characterized by a mean molecular mass of between 1000kg/mol and 4000 kg/mol, even more particularly between 1000 kg/mol and 2000 kg/mol.

Alternatively, the molecular mass of the hyaluronan and/or hyaluronan peptide conjugates provided herein are characterized by the number n of the hyaluronan disaccharide base unit. In certain embodiments, n is ≥2.500. In certain embodiments, n is between 2.500 and 10.000, particularly between 2.500 and 5.000.

These high molecular weights distinguish the processes and gels provided herein from those known in the art, which have been obtained with hyaluronan derivatives having distinctly lower mean molecular mass. This difference leads to significant advantages both in making, handling and applying the gels provided herein. These advantageous qualities include the size stability and gel formation kinetics.

Where a molecular weight value is used herein, it is to be construed as determined by static light scattering (SLS), particularly as implemented as multi-angle light scattering (MALS) in series after gel permeation chromatography (GPC) or size exclusion chromatography (SEC) (as described in Mendichi and Schieroni, Bioconjug Chem. 2002 Nov-Dec;13(6):1253-8).

In certain embodiments of any process provided herein (1^{st} and 2^{nd} aspect), the transglutaminase donor peptide sequence is selected as follows:

Most generally, a lysine donor (TG donor peptide) can be characterized as a small chemical group (less than 3 kDa) containing a primary amine as well as a thiol (the thiol being the group undergoing Michael addition to the divinylsulfone moiety). Particularly suitable are transglutaminase donor peptides of 1 to 30 amino acid length that contain a K (lysine) and a C (cysteine). More specifically, peptides containing a cysteine and the sequence XKG where X is a hydrophobic amino acid such as L (leucine), I (isoleucine), V (valine), F (phenylalanine), Y (Tyrosine), W (Tryptophan) or DOPA (3,4-Dihydroxy-L-phenylalanine) and/or G is glycine, are good substrates for FXIIIa; even more particularly, this is true of peptides containing the amino-terminal sequence Ac-XKG where Ac- is an acetylate group. Specific examples of exemplary donor peptides are peptides that comprise, or essentially are/consist of, the sequences Ac-FKGGERCG-NH₂ (SEQ ID NO 01), Ac-FKGGC-NH2 (SEQ ID NO 02), Ac-FKGGERCG (SEQ ID NO 03), Ac FKGGC (SEQ ID NO 04), Ac-LKGGC (SEQ ID NO 05), Ac-DOPA-KG-C (SEQ ID NO 06), Ac-FKGG-GPQGIWGQ-ERCG-NH2 (SEQ ID NO 07), Ac-FKGG-GPQGIAGQ-ERCG-NH2 (SEQ ID NO 08), Ac-FKGG-GPQGIWGQ-C (SEQ ID NO 09), Ac-FKGG-GPQGIAGQ-C (SEQ ID NO 10), Ac-FKG-C-NH2 (SEQ ID NO 11), Ac-FKG-C (SEQ ID NO 12), Ac-LKG-C-NH2 (SEQ ID NO 13), Ac-LKG-C (SEQ ID NO 14).

In certain embodiments of any process provided herein (1^{st} and 2^{nd} aspect), the transglutaminase acceptor peptide sequence is selected as follows:

Most generally, a glutamine acceptor (TG acceptor peptide) can be characterized as a small chemical group (less than 3 kDa) containing a terminal amide as well as a thiol (the thiol being the group undergoing Michael addition to the divinylsulfone moiety). Particularly suitable are transglutaminase acceptor peptides of 1 to 30 amino acid length that contain a Q (glutamine) and a C (cysteine). More specifically, peptides containing a cysteine and a sequence selected from QQ, QL, QE and a C are good substrates for FXIIIa; even more particularly, this is true of peptides containing the sequence NQEQVSPL (SEQ ID NO 15), QQLG (SEQ ID NO 16) or GQLKHLEQQEG (SEQ ID NO 17) and a cysteine. Specific examples of exemplary acceptor peptides are peptides that comprise, or essentially are/consist of, the sequences NQEQVSPLC (SEQ ID NO 18), NQEQVSPL-ERCG (SEQ ID NO 19), NQEQVSPL-GPQGIWGQ-ERCG (SEQ ID NO 20), NQEQVSPL-GGG-ERCG (SEQ ID NO 21), NQEQVSPL-DRCG (SEQ ID NO 22), Ac-GQQQLG-C-NH2 (SEQ ID NO 23), GQQQLG-ERCG (SEQ ID NO 24), GQQQLG-DRCG (SEQ ID NO 25), GQQQLG-C (SEQ ID NO 26), GQLKHLEQQEG-C (SEQ ID NO 27), GQLKHLEQQEG-ERCG (SEQ ID NO 28), GQLKHLEQQEG-DRCG (SEQ ID NO 29) wherein each of the acceptor sequences can show an N-acetyl or a H- at the N-terminus, and a NH₂- or OH- at the C-terminus.

Another (third) aspect of the invention relates to a composition comprising / essentially consisting of a hyaluronan polymer of general formula I, 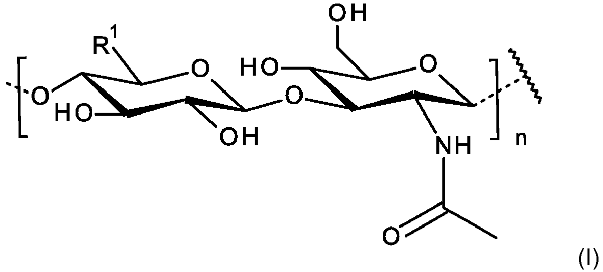 wherein 5% to 20%, particularly 8-12%, more particularly approximately 10% of R¹ moieties are represented by a general formula II, 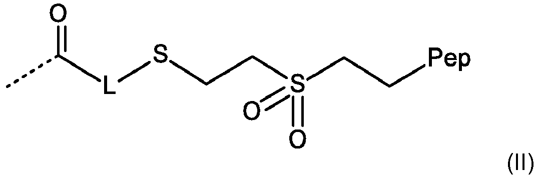 wherein
- L is a linker moiety having a molecular mass of ≤150g/mol, particularly a linker consisting of 2, 3, 4, 5 or 6 C, N and/or O atoms in the linking chain (plus additional H as applicable), more particularly L is NH-Alk or O-Alk or NH-NH-CO-Alk with Alk being a C1 to C5 alkyl, even more particularly L is -N-NHCO-(CH₂)₂-, and
- Pep is a transglutaminase donor peptide or a transglutaminase acceptor peptide, respectively, and the rest of R¹ moieties are represented by COOH.

Any donor and / or acceptor peptide as defined above may be employed in practicing the invention as defined by any of the aspects or embodiments thereof recited above or below.

An alternative of this third aspect of the invention relates to a composition comprising or essentially consisting of high molecular weight hyaluronan modified by
i. thiolation of 5% to 20%, particularly 8-12%, 10% of COOH functions comprised in the hyaluronan
ii. Michael addition of divinyl sulfone on thiol moieties introduced in step i.,
iii. Michael addition of cysteine containing peptides to vinyl moieties introduced in step ii.

In certain embodiments, the composition is characterized by a mean molecular weight equal or greater than (≥) 250 kg/mol, ≥ 500 kg/mol, particularly greater than 1000 kg/mol (1 MDa); more particularly between 1.000.000 g/mol and 4.000.000 g/mol, even more particularly between 1.000.000 g/mol and 2.000.000 g/mol.

Alternatively, the composition may be characterized with respect to its mean molecular weight by the number n of the hyaluronan disaccharide base unit in the polymer chain (see formula I). In certain embodiments, n is ≥2.500. In certain embodiments, n is between 2.500 and 10.000, particularly between 2.500 and 5.000.

In certain embodiments, the composition according to this third aspect of the invention, or any of the embodiments contemplated thereof, further comprises a sulfated polysugar, particularly a sulfated polysugar selected from alginate sulfate, hyaluronan sulfate, chondroitin sulfate, fucan sulfates (1->3-linked alpha-L-fucopyranose oligomers with a certain degree of sulfate substitution at the 2- and 4-positions, see US 6720419 B2 and documents cited therein, all of which are incorporated by reference herein), carrageenans, ulvans (sulfated xylorhamnoglucuronan), heparin and heparan sulfate, particularly at a ratio of 2,5% to 15% (m/m), more particularly at a ratio of 5% to 10% (m/m) relative to the mass of hyaluronan polymer..

In certain particular embodiments, the composition according to this third aspect of the invention, or any of the embodiments contemplated thereof, further comprises heparin or heparan sulfate, particularly at a ratio of 2,5% to 15% (m/m), more particularly at a ratio of 5% to 10% (m/m) relative to the mass of hyaluronan polymer. The heparin or heparan sulfate may either be comprised covalently linked to the HA gel, or as a freely diffusible addition.

In certain embodiments, the heparin or heparan sulfate comprises covalently attached transglutaminase donor and / or acceptor peptides, particularly wherein 10% to 20% of carboxylic acid groups present in said heparin or heparan sulfate are covalently modified, more particularly covalently modified to contain a modification described by general formula (II) as laid out above [wherein the carbon shown on the left is the carboxylic carbon comprised within the glucosaminoglycan backbone], with L, S and Pep having the meaning indicated above.

In certain embodiments, the composition according to this third aspect of the invention, or any of the embodiments contemplated thereof, is characterized in that it comprises a transglutaminase donor peptide comprising or essentially consisting of a sequence selected from any one of SEQ ID NO 01 to SEQ ID NO 14.

In certain embodiments, the composition according to this third aspect of the invention, or any of the embodiments contemplated thereof, is characterized in that it comprises a transglutaminase acceptor peptide comprising or essentially consisting of a sequence selected from any one of SEQ ID NO 15 to SEQ ID NO 29.

In certain embodiments, the composition according to this third aspect of the invention, or any of the embodiments contemplated thereof, is characterized in that it comprises a hyaluronan polymer as specified above, comprising a transglutaminase acceptor peptide comprising or essentially consisting of a sequence selected from anyone of SEQ ID NO 15 to SEQ ID NO 29, and a hyaluronan polymer as specified above, comprising a transglutaminase acceptor peptide comprising or essentially consisting of a sequence selected from any one of SEQ ID NO 15 to SEQ ID NO 29, wherein the donor and acceptor peptides are present in approximately equal molar amounts.

In certain embodiments, the composition according to this third aspect of the invention, or any of the embodiments contemplated thereof, is characterized in that it comprises a hyaluronan polymer comprising transglutaminase donor peptides and a hyaluronan polymer comprising transglutaminase acceptor peptides, wherein donor and acceptor peptides are present in approximately equal molar amount, and the composition further comprises a Factor XIII polypeptide and / or a thrombin polypeptide.

A preferred presentation for that type of compositions is that where the two distinct hyaluronan polymers and (one or two) enzyme(s) are separated in two or three stocks in any of the combinations preventing an initiation of the gel cross-linking reaction prior to their mixing. Non exhaustive examples of such permutations are: HA-TG/Lys and thrombin polypeptide in one stock, HA-TG/Gln and FXIII polypeptide in the other stock, HA-TG/Lys and HA-TG/Gln in one stock and activated FXIII polypeptide in the other stock, HA-TG/Lys HA-TG/Gln and thrombin polypeptide in one stock, FXIII polypeptide in the other stock, HA-TG/Lys and HA-TG/Gln in one stock, FXIII polypeptide in another stock, Thrombin polypeptide in a third stock. The polymers and enzymes stocks might be in liquid solution, in frozen solution, or in lyophilized form. For convenience of use, the stocks might be loaded in a double barrel syringe equipped with a static mixer or with converging needles for mixing to occur during delivery. They might also be combined with single barrel syringes or micropipettes and pre-mixed manually or left to mix in situ by diffusion.

In certain embodiments, the composition according to this aspect of the invention is provided in dried form.

In certain embodiments, the composition according to this third aspect of the invention, or any of the embodiments contemplated thereof, is characterized in that it comprises a hyaluronan polymer comprising transglutaminase donor peptides and a hyaluronan polymer comprising transglutaminase acceptor peptides, wherein donor and acceptor peptides are present in approximately equal molar amount, and the composition further comprises a thrombin polypeptide, but no factor XIII polypeptide, nor calcium.

In certain embodiments, the composition according to this third aspect of the invention, or any of the embodiments contemplated thereof, comprises hyaluronan polymer comprising transglutaminase donor peptides and a hyaluronan polymer comprising transglutaminase acceptor peptides. The composition further comprises thrombin to result in a ratio of thrombin to total HA polymer (the sum of both HA polymers) of between 300 U thrombin per gram HA polymer (leading to an activity of 3 U/ml for a 1% gel, and 9 U/ml for a 3% gel) and 1500 U per gram HA polymer (leading to an activity of 15 U/ml for a 1 % gel, and 45 U/ml for a 3% gel). Particularly useful for surgical applications are compositions comprising approx. 1000 to 1500 U thrombin per gram HA polymer (leading to concentrations of 10 to 15 U per ml in a 1% gel).

Another aspect of the invention relates to a hyaluronan hydrogel comprising transglutaminase cross-linked hyaluronan and water, obtained by a process according to the first aspect of the invention, or any of its specific embodiments.

Alternatively, this aspect of the invention may similarly framed as providing a hyaluronan hydrogel obtained by crosslinking a hyaluronan polymer modified by the process according to the second aspect of the invention, or by crosslinking a composition as provided here.

In certain embodiments, the hyaluronan hydrogel comprises 0,25% to 0,99% of cross-linked hyaluronan in water (w/v), particularly 0,25% to 0,75% (w/v), or 0,4% to 0,6% (w/v), particularly ca. 0, 5% (w/v).

In certain embodiments, the hyaluronan hydrogel comprises 0,5% to 4% of cross-linked hyaluronan in water (w/v), particularly 1% to 2% (w/v).

In certain embodiments, the hyaluronan hydrogel additionally comprises 0,01% to 0,5% (w/v) of heparin or heparan sulfate cross-linked to the hyaluronan, particularly 0,05% to 0,2% (w/v).

In certain embodiments, the hyaluronan hydrogel, particularly the hydrogel that additionally comprises heparin or heparin sulfate, comprises a growth factor selected from transforming growth factor beta 1, 2, or 3, insulin like growth factor 1 and fibroblast growth factors 1, 9, or 18.

In certain select embodiments thereof, the concentration of said growth factor is between 10 ng/ml and 3000 ng/ml. In certain embodiments, the gel comprises a growth factor selected from transforming growth factor beta 1, 2, or 3 at a concentration of between 10ng/ml and 100 ng/ml. In certain embodiments, the gel comprises a growth factor selected from transforming growth factor beta 1, 2, or 3 at a concentration of between 1500ng/ml and 2500 ng/ml.

In certain embodiments, the hyaluronan hydrogel comprises is characterized by a pore size of between 0.1 µm and 1 µm, particularly between 0.2 and 0.8 µm.

The pore size of the HA-TG formulation preferentially used for neural cultures was evaluated with confocal imaging, using a small amount (-50 µmol/L) of TG/Gln-Fluorescein co-cross-linked with the polymer as a fluorescent reporter. Pores in the range of 0.2 to 0.8 µm could be visualized. Single plane confocal imaging of an HA-TG 0.5% (w/v) hydrogel fluorescently tagged with TG/Gln-Fluorescein. Pores in the range of 0.2 to 0.8 µm can be distinguished.

In certain embodiments, the hyaluronan hydrogel is characterized by an adhesion strength, as measured by push-out assay, of more than 0.5 kPa, in particular 1 to 10 kPa, more particularly around 2 kPa when adhesion to a cartilage surface is measured.

In certain embodiments, the hyaluronan hydrogel is characterized by its adhesion strength, as measured by push-out assay, of more than 5kPa, particularly approx. 6 kPa when adhesion to a chondroitinase treated cartilage surface is measured. The push-out assay is described in Example 1. Chondroitinase treated cartilage is prepared according to Hunziker + Kapfinger (1998) J Bone Joint Surg Br 80:144-150.

In certain embodiments, the hyaluronan hydrogel is characterized by a change in dimension (swelling or shrinking), as measured by comparing gel disk diameters after gelation and after additional swelling in an isotonic aqueous buffer for 3 days, of less than 20% on average, in particular less than 10% on average, more particularly less than 4%. In certain embodiments, the change in dimension is determined on average with a standard deviation of less than 10%, where the aqueous buffer is an aqueous solution approximating physiological conditions including but not restricted to physiological saline, phosphate buffered saline, cell culture media, blood serum or synovial fluid.

According to certain embodiments, the hyaluronan hydrogel as provided herein additionally comprises chondrogenic cells as defined in that such cells express collagen 2 and aggrecan, or are able to give progeny expressing collagen 2 and aggrecan, particularly chondrocytes, chondroprogenitors, mesenchymal stem cells or adipose stem cells.

According to certain embodiments, the hyaluronan hydrogel as provided herein additionally comprises neurons, neural cells and/or neuroprogenitor cells (particularly primary or induced pluripotent stem cells, or embryonic stem cells or neural progenitor cells, neurons and/or oligodendrocytes and/or astrocyte mono or co-culture. In certain embodiments said cells are derived from species including but not restricted to primates, rodents, avians; more particularly primary neurons from mouse or rat and induced pluripotent stem cell derived human neurons). Particularly favourable conditions for cell embedding and culture are provided by gels having a concentration of hyaluronan-peptide conjugate in aqueous solution of 0.25 to 1.5% (w/v), particularly 0.35 to 1% (w/v), more particularly ∼0.5% (w/v).

In certain embodiments, the cell density of such embedded cell gels is 0.1 to 100 million cells/ml, particularly 0.5 to 50 million cells/ml, more particularly 1 to 15 million cells/ml.

In certain embodiments, the hyaluronan hydrogel provided herein further comprises particles and/or fibres derived from cartilage. In certain embodiments, said particles consist of, or comprise, tissue particles. In certain embodiments, said particles consist of, or comprise, cartilage particles. In certain embodiments, said particles consist of, or comprise, particles consisting of lyophilized cartilage tissue. In certain embodiments, said particles consist of, or comprise, human cartilage tissue. In certain preferred embodiments, said particles consist of, or comprise, autologous cartilage tissue. In certain preferred embodiments the particles can be clinical products of micronized matrix including BioCartilage, Amniofix, Alloderm-Cymetra, Cook Biotech Small Intestial Muscosa (SIS) particles. In certain preferred embodiments the particles can be hydroxyapatite or calcium phosphate.

In certain embodiments, the particles and/or fibres are made of a synthetic polymer, particularly a polymer selected from the group consisting of polymers, or polymers derived from, polyethylene glycol, polypropylene glycol, gel forming poloxamers F108, F127, F68, F88, polyoxazolines, polyethylenimine, polyvinyl alcohol, polyvinyl acetate, polymethylvinylether-co-maleic anhydride, polylactide, poly-N-isopropylacrylamide, polyglycolic acid, polymethylmethacrylate, polyacrylamide, polyacrylic acid, and polyallylamine or co-polymers of these or block-copolymers of these.

In certain embodiments, the particles and/ or fibres comprise or are predominantly or exclusively composed of minced tissue. In certain embodiments, the minced tissue is derived from tissue selected from the group consisting of auricular cartilage, nasal cartilage, nucleus pulposus, meniscus, trachea, nasal cartilage, rib cartilage, articular cartilage, synovial fluid, vitreous humor, brain, spinal cord, muscle, connective tissues, small intestinal submucosa and liver. In certain embodiments, the minced tissue is in the range of from 5 µm - 50 µm, 50 - 200 µm and 200 - 1000 µm or a combination of these.

In certain embodiments, the particles can contain living cells.

In certain embodiments, cells are grown ex-vivo in the HA hydrogels of the invention, for later implantation into a suitable site in the body.

In certain other embodiments, the HA hydrogels of the invention are used together with cells in a formulation where the gel precursor composition is mixed with the cells and injected into the patient prior to gel formation, so that the cells are embedded in the gel *in situ.* Remarkably, the inventors have found that HA hydrogels having a HA concentration of 0,5% to 2% (w/v) enable chondrogenic cells to spread, proliferate and deposit a homogeneous matrix with the result that the stiffness of the gels can go from ∼1 kPa up to >100 kPa after implantation. This is in contrast to the HA gels known in the art, where cells do not spread, proliferate little or not at all, and deposit a ring of pericellular matrix that does not give rise to any significant increase in stiffness.

The gels provided herein are distinguished from gels known in the art by useful and unusual properties. In particular, the HA hydrogels provided herein can be tuned so not to swell or shrink for concentrations ranging at least from 1 to 3%. This quality is also referred to as shape-stability or form-stability herein, and it is shown in the mechanical characterization figure of Example 1 (no mass loss and no change in swelling ratio). This property is important, for example, when the hydrogel is used in vivo, e.g. in treatment of a cartilage defect, because it will help the gel stay in place.

In certain embodiments of the hydrogel provided herein, the ratio of the gel disk diameters before and after swelling in PBS for 3 days was of 0.99+/-0.10 for 3% gels (SD n=8), 1+/- 0.025 for 2% gels (SD n=3) and 1.03+/-0.05 for 1 % gels (SD n=3).

Without wanting to be bound by theory, the inventors believe that the linker definitely plays a role in this shape stability, as further elaborated upon below in section 2.3 of Example 1.

In one aspect, the invention provides a novel injectable hydrogel with ideal properties for one-step cartilage repair procedures. Many gels supporting chondrocytes in 3D cultures have been proposed, and the gold standards in vitro are agarose and alginate, but since these gels do not adhere to cartilage surface, fibrin is still almost always the choice in a surgical setup, despite being very short lived, and promoting scar formation rather than hyaline cartilage regeneration.

Example 1 below shows one example of the transglutaminase-crosslinked hyaluronan hydrogels (HA-TG) provided herein, which are simultaneously injectable, mitogenic, chondroinductive, and strongly adhesive to native cartilage. Human chondroprogenitors are encapsulated in HA-TG gels and their growth and chondrogenesis is tunable based on macromere content. Strikingly, within the softest 1% gels (∼1 kPa), chondroprogenitors proliferate and deposit extracellular matrix to an extent that the hydrogels reach a modulus (-0.3 MPa) approaching that of native cartilage (∼1 MPa) within 3 weeks. HA-TG hydrogels are fully biocompatible and provide an excellent mimic of the native cartilage extracellular matrix, being recognized by multiple cell types. Used in combination with off-the-shelf human chondroprogenitor cells, HA-TG hydrogels lay the foundation for a cell-based treatment for cartilage lesions which is minimally invasive, highly reproducible, and achieves integration with the surrounding tissue.

In comparison to the gel published by Ranga et al. (ibid.), the formulation of the present invention forms stable gels down to <0.25% (w/v), which is important for neuron cultures, which need an extremely soft but stable matrix, and applications in orthopedical surgery. The gels of the invention are shape-stable (they do not swell or shrink) over a range covering at least 1 to 3% (w/v), which is a rare and important property for cartilage regeneration, for the gels not to delaminate or 'pop out' (bulge or dislocate) after they are formed in a knee defect.

Advantages in neural applications:

The inventors herein demonstrate for the first time HA gels encapsulated neurons which were highly viable, quickly extended neurites in 3D, specified axons and dendrites, formed active synapses, and showed long-term stable and coordinated spiking activity.

Hydrogels transglutaminase (TG) cross-linked high MW HA gels have significant advantages for neural tissue engineering compared to previous HA gels. Due to their chemical inertness in the absence of FXIIIa, the material can be stored long-term, is stable in solution, and shows no cytotoxicity. The gelation is significantly more cell-friendly than gels known in the art, due to the specificity of the enzyme. The gelation rate can be tuned by adjusting the amount of FXIIIa added, and can be made less than 40 seconds. The gels are injectable, and attach covalently to fibrinogen and fibrin, two common bioactive components in *in vitro* tissue engineering, as well as proteins present *in vivo,* allowing the gels to covalently bind to brain or spinal cord defects. These optimal chemical and bioactive properties of HA-TG gels enabled the formation of 3D neuronal cultures of unprecedented performance, showing fast neurite outgrowth, axonal and dendritic speciation, strong synaptic connectivity in 3D networks, and rapidly-occurring and long-lasting coordinated electrical activity.

Wherever alternatives for single separable features such as, for example, a gel concentration, acceptor or donor sequence or cell type are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Brief description of the Figures

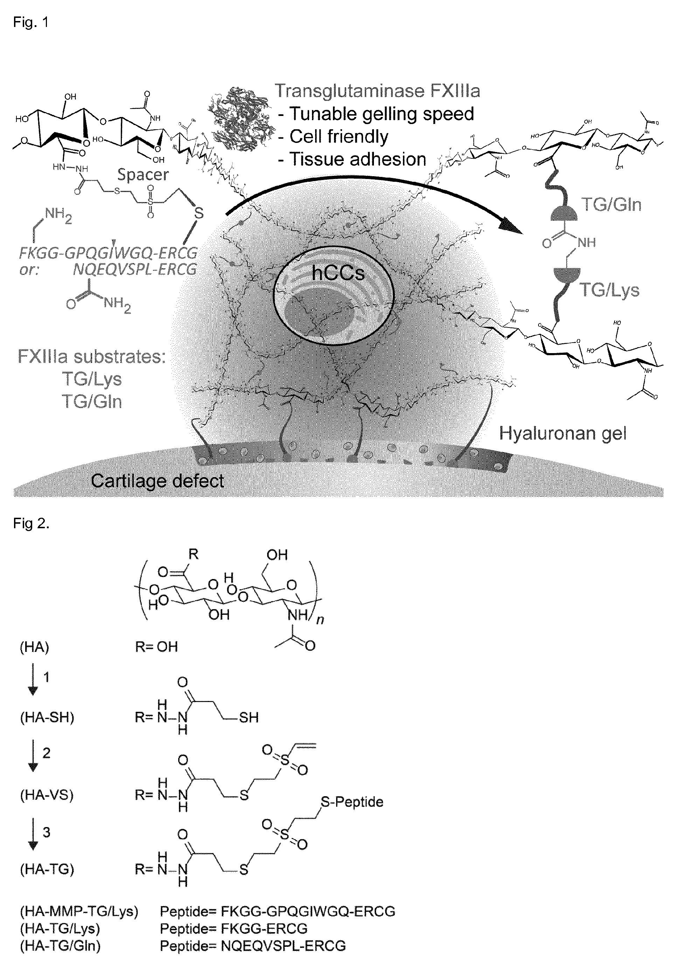

- Fig. 1: shows concept schematics of the gelling mechanism of transglutaminase (TG) cross-linked hyaluronan (HA). The lysine and glutamine residues that are covalently cross-linked by the TG FXIIIa are highlighted on the left, and the amide bond resulting from the conjugation is shown on the right. HA chains appear in black, peptides in red with reactive side chains explicitly shown, and the spacer/adapter between the peptides and the HA is in purple. Encapsulated human chondroprogenitor cells (hCCs) and adhesion to cartilage tissue are also represented.
- Fig. 2: shows the chemical synthesis of HA-TG precursors. Reaction conditions are 1/ EDC, MES pH4.1 to 4.5, DTPHY then TCEP, 2/ DVS, TEOA pH8, 3/ Peptide, TEOA pH8.

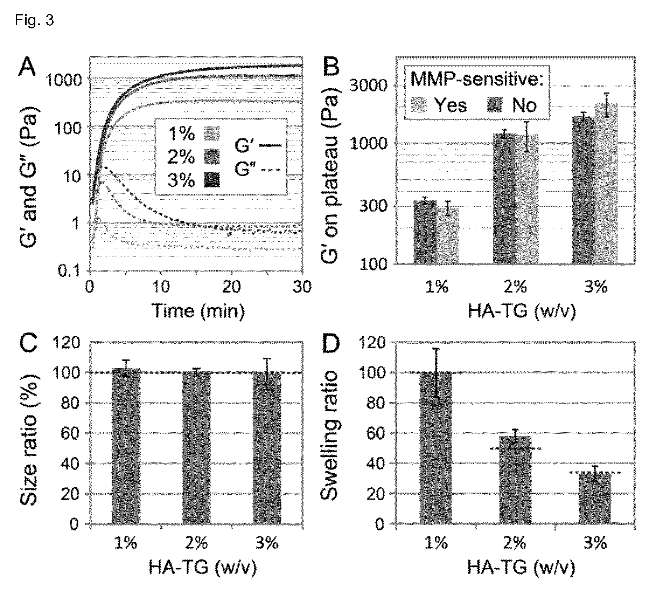

- Fig. 3: shows the material properties of gels corresponding to Example 1. (A) Representative gelling curves as monitored by rheometry showing the build-up of the storage G' and loss G" moduli for various concentrations of HA-TG. Note the fast gelation and equilibration to a plateau. (B) Values of the final storage modulus (taken at 30 min), showing the range of stiffness used in this study and the absence of influence from MMP sensitive sequence addition. (C) Change in diameter of the gels after 4 days of swelling in PBS. The dotted line indicates no change. (D) Swelling ratio (wet/dry weight of the gels) after 4 days of swelling in PBS. The dotted line indicates the values expected for no mass loss nor volume change. Error bars: SD with n=8 (for C and D at 3%) or n=3 (the rest).

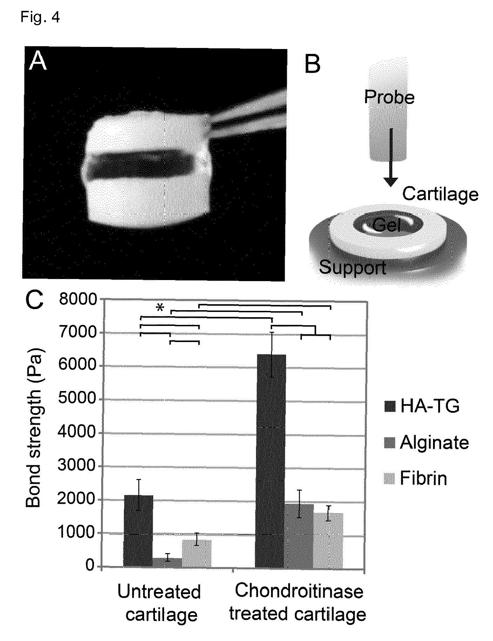

- Fig. 4: shows an example of a gel according to Example 1 and its adhesion to cartilage. (A) Cartilage biopsies glued together with a gel layer. (B) Setup of the push-out assay for bond strength determination. (C) Bond strength of HA-TG gels compared with alginate and fibrin. (*) p<0.05. Error bars: SEM n=6.

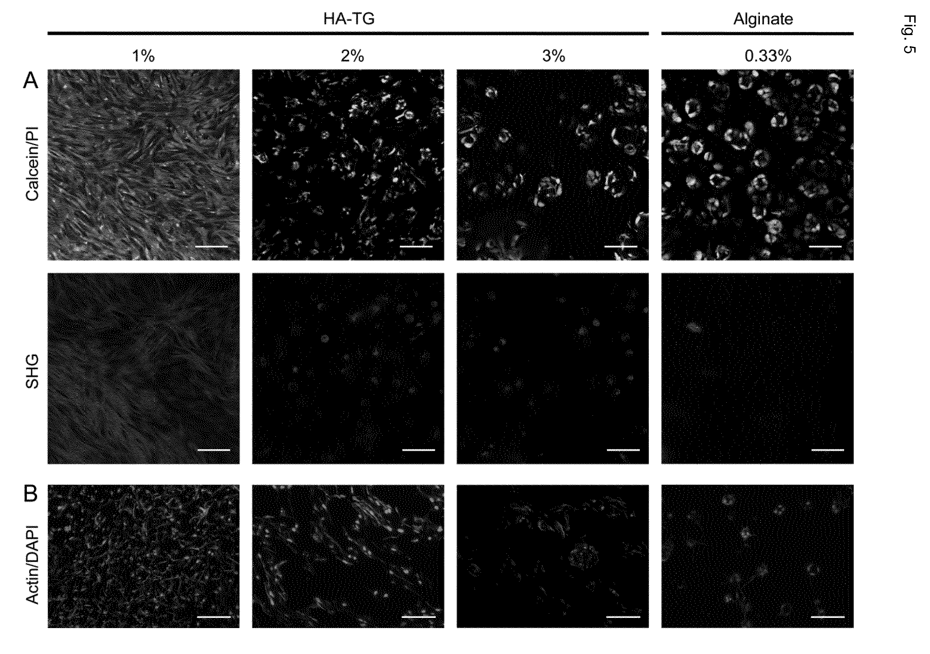

- Fig. 5: shows cell and collagen morphology of gels made according to Example 1 visualized with non-specific markers. (A) Multiphoton live imaging, with simultaneous acquisition of calcein (cytoplasm of live cells, green), propidium iodide (PI, nuclei of dead cells, red), and second harmonic generation (SHG, assembled collagen, blue). All conditions are highly viable, while cells spread, proliferate, and deposit a dense collagen matrix in soft HA-TG gels only. Images are at -60 µm depth under the surface of the gels. (B) Actin cytoskeleton (phalloidin, red) and nuclei (DAPI, blue) imaged in the center of the gels from fixed cryosections. This highlights the spreading and proliferation of human chondroprogenitorss in soft HA-TG gels are not merely a surface effect. (A+B) Images are at 21 days post encapsulation. Scale bars: 80 µm.

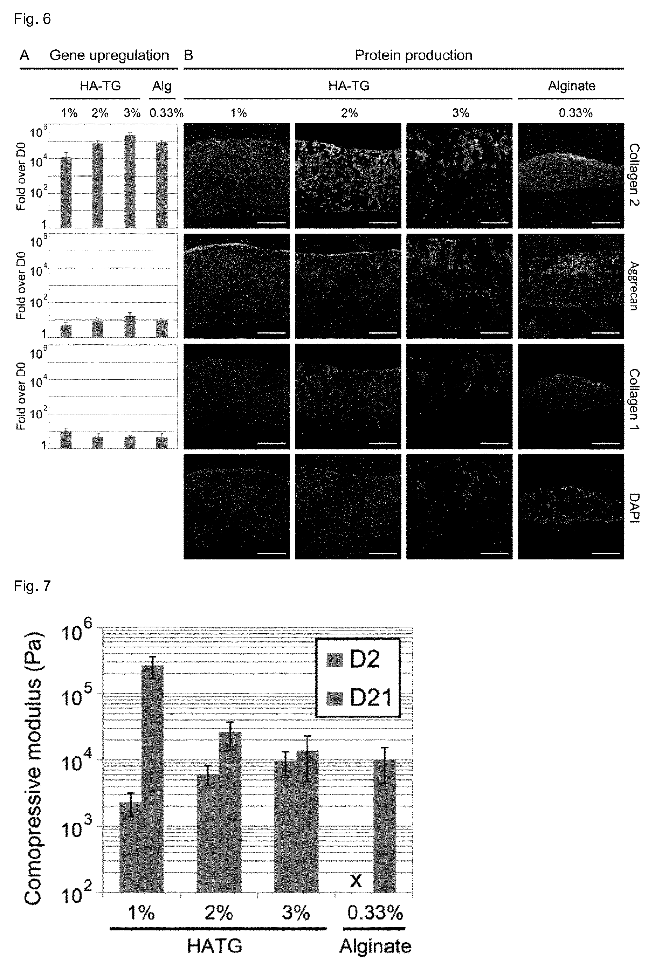

- Fig. 6: shows cartilage production by encapsulated hCCs as seen from (A) gene expression 21 days post encapsulation, normalized to expression on the day of encapsulation, and (B) matrix proteins deposition. Each image is from a vertical slice covering the gel from the bottom to the top around its center. Collagen 1, collagen 2 and DAPI are acquired on the same section. Scale bars: 400 µm.
- Fig. 7: shows the evolution of the mechanical properties with matrix deposition by the hCCs between 2 and 21 days after encapsulation. Bovine hyaline cartilage was found to have a compressive modulus of 10⁶ Pa in the same measuring conditions. Error bars: SD from n=3 (D2) and n=6 (D21).

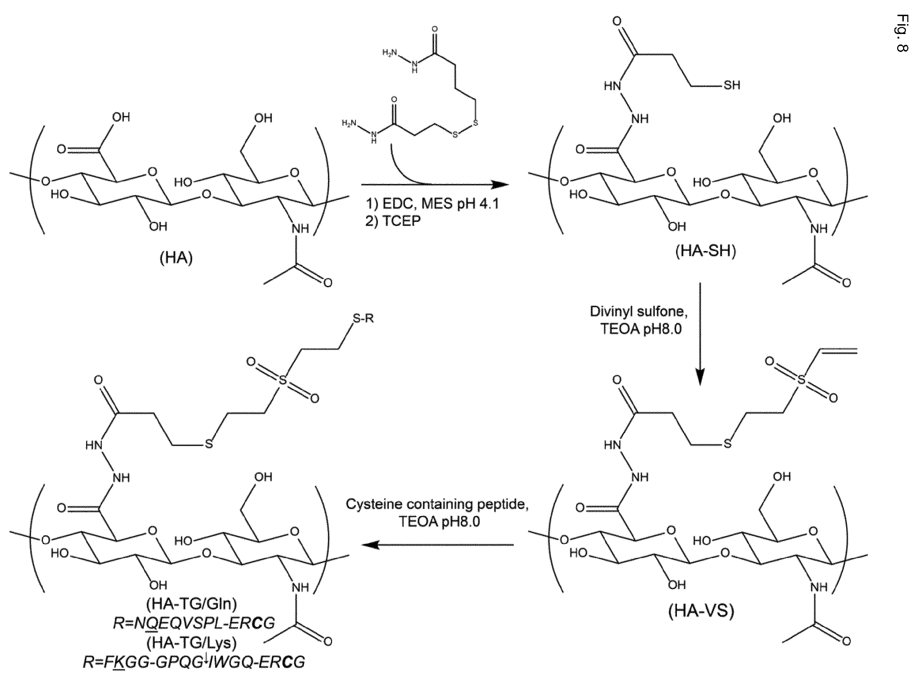

- Fig. 8: shows synthesis of the HA derivatives elaborated upon in Example 2. In the TG peptides, the cysteines that provide thiols for conjugation onto HA-VS are in bold and the lysine and glutamine covalently coupled to each other on their side chains by FXIIIa are underlined. The MMP cleavage site is marked by an arrow.

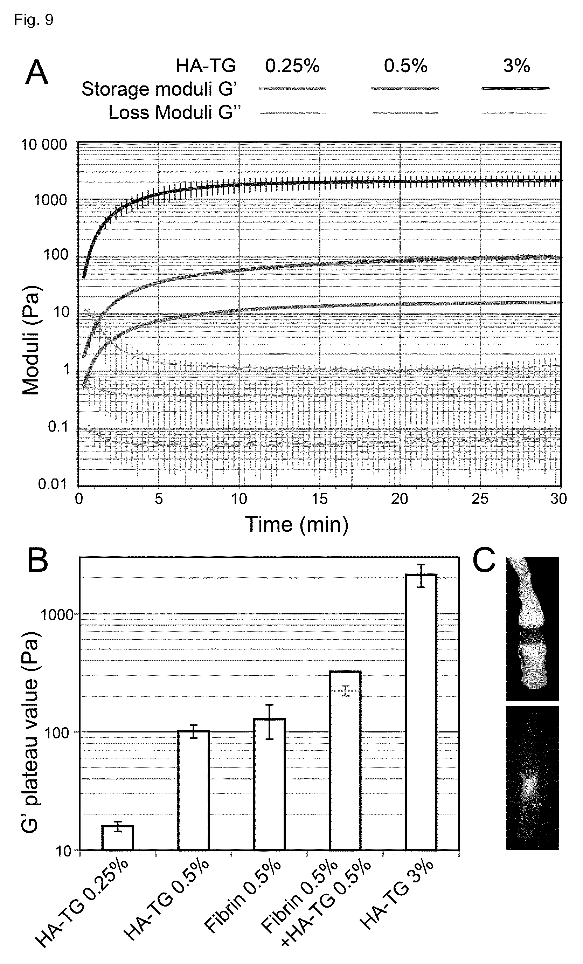

- Fig. 9: shows *the results of mechanical measurements* of *gels made according to Example 2. (A) Gelling profiles for different concentrations of HA-TG* as *monitored by rheometry and (8) Values* of *the storage modulus after* 30 *min (plateau), showing fibrin gels and HA-fibrin hybrids. The dashed line on the HA-fibrin hybrid gel shows the expected modulus if there would be no co-cross-linking or interactions between the HA and fibrin networks. Enzyme concentration was adjusted to keep similar gelation speed for all conditions, respectively in U*/*ml:* FXIIIa *10 for HA-TG 0.25%,* FXIIIa *7.5 for HA-TG 0.5%, FXIII 20 and thrombin 12.5 for HA-TG 3%,* FXIIIa *7.5 and thrombin 0.5 for Fibrin and Fibrin+HA-TG. Error bars: SD from* 3 *to 4 replicates. (C) Demonstration of gel adhesion to mouse spinal cord tissue ex vivo. The tissue is hanging from* a *forceps visible on top and the middle fluorescent segment is the gel.*

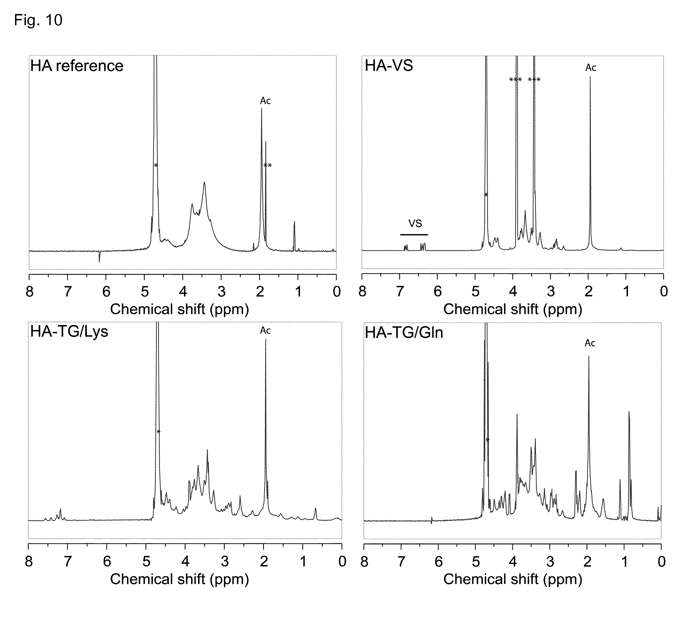

- Fig. 10: shows NMR spectra of the HA starting material of the gels according to Example 2 and the VS and TG substrate derivatives. Note the presence of vinyl protons between 6 and 7 ppm on HA-VS, and their complete disappearance after peptide conjugation, showing complete substitution. The N-acetylate from HA backbone (Ac) is used as an internal reference to quantify degrees of substitution with VS. (*) water (**) acetone (***) TEOA buffer.

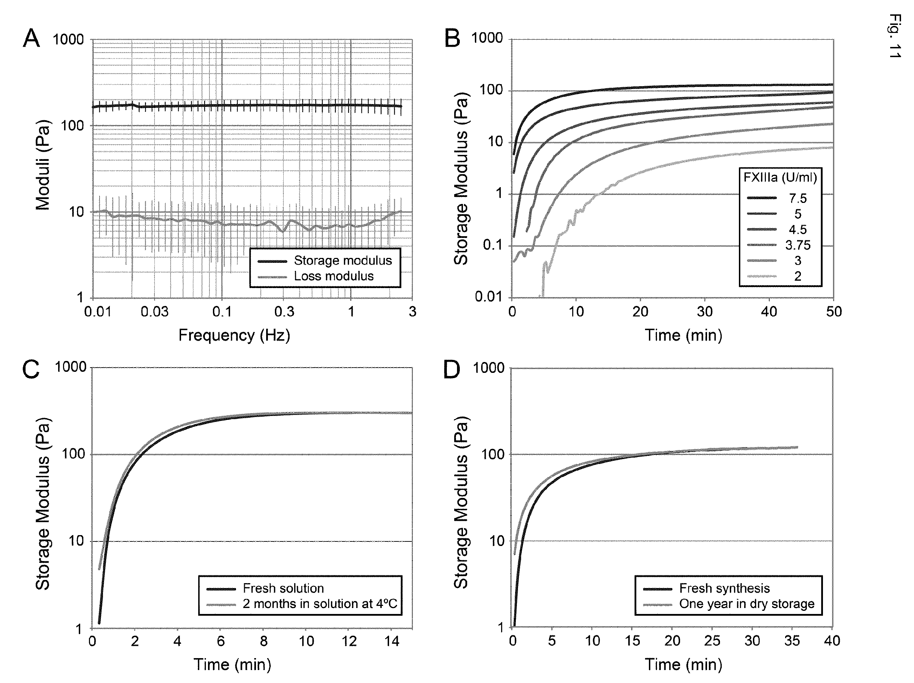

- Fig. 11: shows the results of additional mechanical measurements of the gels according to Example 2. (A) Frequency response of a 0.5% HA-TG gel after free swelling for 3 days in PBS. Error bars: SD n=3. (B) Influence of the concentration of enzyme on the gelling speed of 0.5% HA-TG gels. (C) Comparison of the gelling curve of 1% HA-TG for a fresh solution versus a solution that has been kept for 2 months showing stability in solution. (D) Comparison of the gelling curve of 0.5% HA-TG for a freshly synthesized HA derivative versus one kept frozen in dry form for 1 year showing stability in storage.

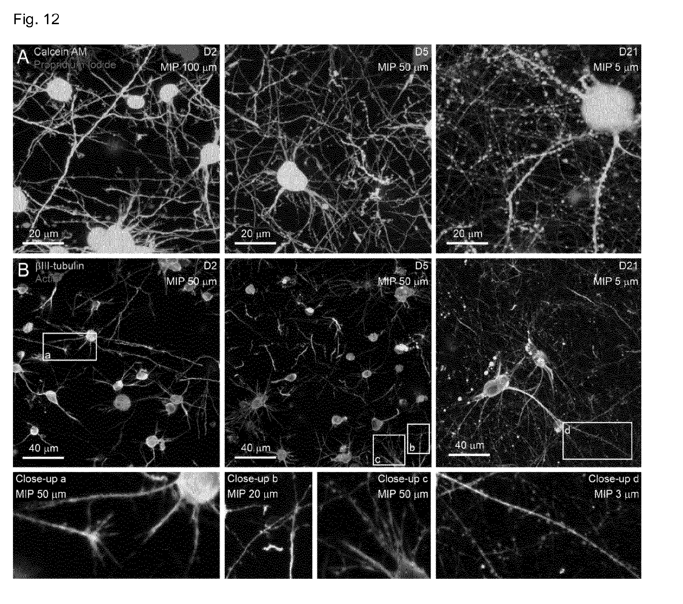

- Fig. 12: shows neuron morphology (A) as seen with calcein staining of all the intracellular space and propidium iodide staining of dead cell nuclei and (B) as seen with cytoskeletal staining. D2, D5, and D21 refer to the number of days neurons spent after encapsulation in the gels. Note the tremendous increase in neurite density overtime as maximum intensity projections (MIP) are done over reduced thickness at later time points. Close-ups emphasize axonal and dendritic growth cones, as well as actin-filled synaptic buttons budding from microtubule-filled dendrites at later time points.

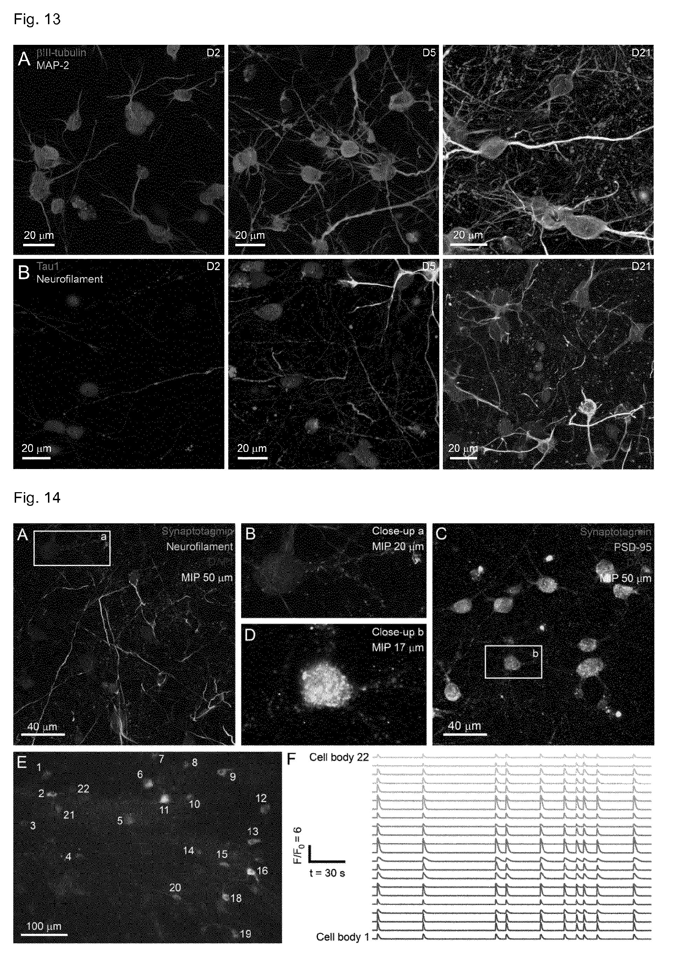

- Fig. 13: shows axonal and dendritic speciation. (A)The mature dendrite marker MAP-2 starts to be expressed in the cell bodies and proximal neurites at D5, and becomes strongly expressed in the whole length of a subset of neurites at D21. The embryonic neuron marker blll-tubulin is present in all neurites. (B) The axonal marker Tau1 is already segregated in some neurites that are therefore axons at D2. By D5, many axons span the gel, and by D21 too many Tau1-positive debris are scattered through the gel to distinguish axons. The mature neuron marker neurofilament starts to be expressed strongly in a few neurons at D5 and is strongly present in a subset of neurites of most neurons at D21 (all neurons have some weak staining at all time points). All pictures are 50 mm MIP.
- Fig. 14: shows synapse formation and coordinated spiking activity. (A) There is a dense mesh of potential presynaptic densities marked by Synaptotagmin (B) the synaptic densities are densely packed on the surface of cell bodies and dendrites (C) There is a similarly dense mesh of potential post-synaptic densities marked by PSD-95 (D) The pre and post synaptic densities are often found in close apposition to each other, showing many synapses are present. (E) Neurons transfected with the genetically encoded calcium reporter GCaMP (F) Measurement of calcium level in the neurons of (E) with high speed confocal microscopy. All neurons in the field of view spike fully synchronously, confirming strong synaptic connectivity. (A-D) are at D21 and (E-F) at D10.

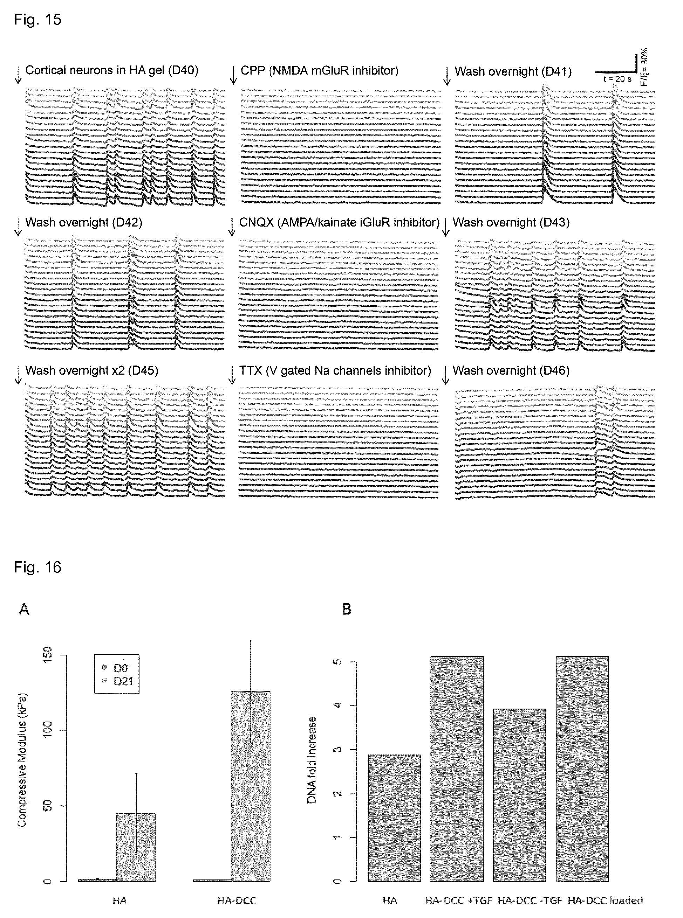

- Fig. 15: shows the results of a characterization of the spiking activity with pharmacological blockers confirms that calcium spikes are associated with neuronal electrical activity and glutamatergic excitation. Each line shows the calcium level in a different cell body from fluorescent reporter imaging, and the arrows indicate media changes, with incubations of 30 min to 1h when adding an inhibitor and up to overnight for washing steps.
- Fig. 16: shows A) Elastic modulus of HA gels with and w/o DCC at day 0 and 21. B) DNA fold increase after 3 weeks in gels without particles (HA), gels with DCC in media with TGF (HA-DCC+TGF), media without TGF (HA-DCC-TGF) and gels with loaded DCC and media without TGF.

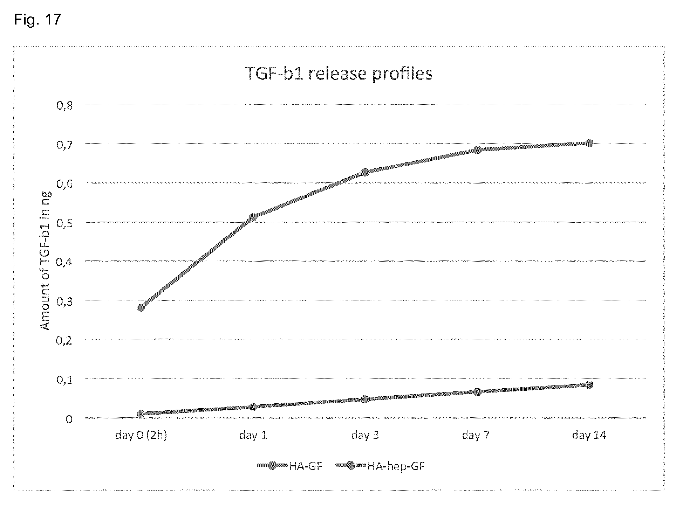

- Fig. 17: shows the release profile of TGF-b1 from HA-TG gels (blue line) and HA-TG + 0.1% Heparin-Gln (orange line)

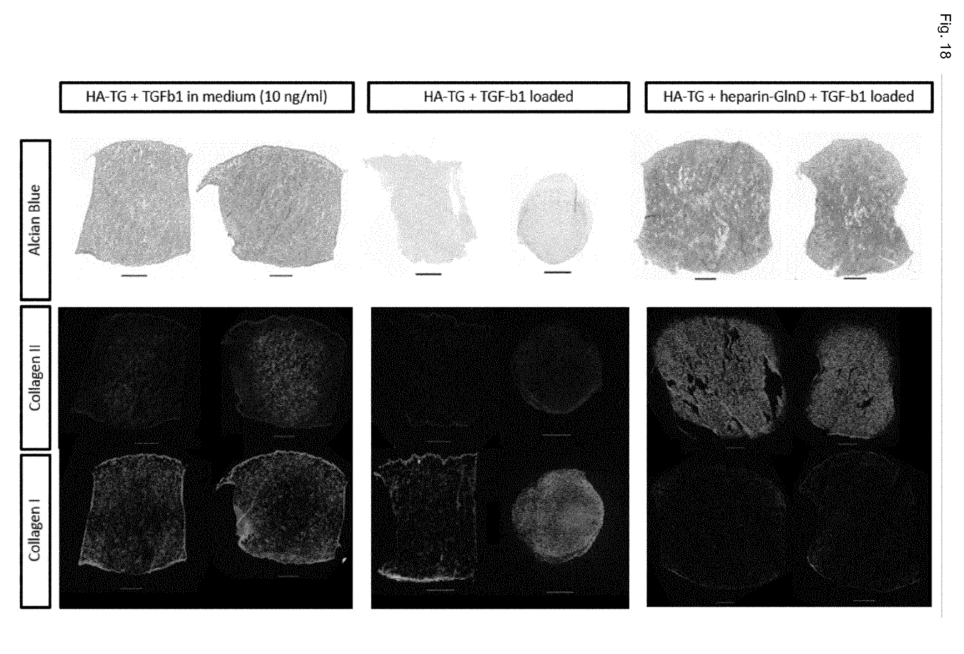

- Fig. 18: shows the histology of gel scaffolds (duplicates) produced with and without heparin. The panels show glycosaminoglycan content (Alcian Blue staining), Collagen II (green) and Collagen I (red). Scale bar: 500 µm.

### Examples

### Example 1: Factor XIII Cross-linked Hyaluronan Hydrogels for Cartilage Tissue Engineering

### 2.1. Material design

To engineer a hydrogel adapted for injection during arthroscopic surgery and supporting cartilage repair, two critical initial choices were made on the polymer backbone and cross-linking strategy.

The two main large polymers in cartilage are HA and collagen 2. Collagen 2 is shielded from the immune system in vivo, so soluble collagen 2 injections give a high risk of triggering antibody production that would ultimately give dramatic cartilage inflammation. HA instead is an ideal scaffold material for cartilage tissue engineering. It is a key non-immunogenic component of the cartilage extracellular matrix, where it fulfils both signalling and structural roles, the latter involving the binding of aggrecan monomers into large aggregates important for load bearing. We used pharmaceutical grade HA of 1 to 1.8 MDa, some of the highest molecular weight on the market, to also benefit from the positive properties of high MW HA. To impart FXIIIa sensitivity to HA, we substituted the polymer with a spacer followed by peptides which are specifically recognized and covalently ligated by the enzyme. One of the peptides is donating the reactive lysine (TG/Lys) and the other the reactive glutamine (TG/Gln). HA substituted with TG/Gln and TG/Lys are synthesized separately, and then combined in equimolar amounts together with the enzyme to trigger the gelation, which can be done directly into cartilage defects and with encapsulated cells, as illustrated in Fig. 1. The HA starting material as well as FXIII and thrombin used are all widespread clinical products, making it unlikely that the gel derivative creates an immune response, and the fast and mild gelation makes the material particularly adapted to injection in situ, which is optimum for clinical translation.

### 2.2. Chemical synthesis

Direct conjugation of peptides onto HA is possible but has several disadvantages. Firstly, the lysine donor peptides can react in an uncontrolled manner through both the side chain amine and the terminal amine. Secondly, EDC-mediated conjugation of carboxylic acids to amines in aqueous conditions has only moderate yields, which is problematic for expensive reagents such as peptides. And most importantly, even after careful high pressure liquid chromatography (HPLC) purification, peptides contain unknown amount of residual salts and trifluoroacetic acid (TFA), which means achieving precise and reproducible substitution rates with different peptide batches is very difficult.

We therefore chose instead to design a new three-step procedure where each reaction is efficient, has no side products and is pushed to completion. This ensures excellent substitution rate reproducibility independent of operator and batches of raw materials, and it also ensures almost no peptide is wasted, making the procedure cost effective.

In short (Fig. 2), HA was first thiolated using EDC activation and hydrazide conjugation of a dithiol containing compound, followed by reduction of the dithiol with TCEP to yield HA-SH. The EDC mediated conjugation is critical as it defines the rate of substitution and therefore the subsequent gelation behavior and mechanical properties of the gel. The use of a buffer rather than the more common manual pH tracking ensures minimal human intervention and therefore great reproducibility. Then, a large excess of divinyl sulfone was added, to exchange with a Michael addition the thiol functionality to vinyl sulfones (VS), yielding HA-VS. Finally, peptides containing a cysteine cassette were reacted onto the HA-VS, using again the efficiency the Michael addition of thiols on VS. At every step, dialysis was the method of choice for removal of the buffers and unreacted small molecules, because it is very efficient with polymers and ensures no stress on the high MW HA chains, preserving the molecular weight. A major motivation for choosing to move on from HA-SH and HA-VS to HA-TG was that thiols get readily oxidized, and very little oxidation was in our experience enough to form a cross-linked sponge when working with very high MW HA. This complicates long-term storage of HA-SH, especially if neutralized. It also negatively affects reproducibility, as solutions of high MW HA-SH in buffer at pH 7.4 undergo significant oxidation even during the few hours of a single experiment. High MW HA-VS, despite of its relatively good stability in solution, has similar storage issues because it hardly resuspends from frozen or lyophilized form, probably because of quickly occurring hydrophobic interactions as well as slow occurring additions of HA hydroxyls or deacetylated amines on the vinyls while in the solid state. For all these reasons, HA-SH and HA-VS were used immediately in the next step. HA-TG derivatives on the opposite had perfect stability in solution, in frozen and in dry form, as checked by rheometry (no change in the gelling curve was found after the polymer had spent >2 months in TBS buffer pH 7.6 at 4°C, or more than a year in dry frozen form, Fig. 11C-D). In this procedure, we targeted (through the amount of reagents in the first step) a substitution rate of 10%, confirmed by proton NMR on the HA-VS in D₂O (VS peaks between 6 and 7 ppm versus acetylate peak from HA backbone at 1.9 ppm, Fig 10). Complete substitution of the HA-VS with peptides was confirmed by the complete disappearance of the VS peaks.

To make gels with MMP sensitivity, a version of HA-TG was made that incorporated an MMP cleavable sequence inside the TG/Lys peptide.

As FXIIIa's native function is to cross-link fibrin clots with themselves and with associated proteins, it is expected that copolymers of fibrin and HA-TG can be formed, as shown in Fig. 9B. The stiffness of a gel containing fibrin and HA is higher than the sum of the stiffness of the fibrin or HA gels alone, showing synergistic interactions are occurring.

### 2.3. Mechanical characterization

The gelling behavior was characterized by rheometry (Fig. 3A-B). Instead of pre-activating FXIII with thrombin like was done in the PEG literature, we reasoned that in situ activation of FXIII would give more interesting gelling kinetics: this way, a quite high amount of FXIII can be used, for very fast equilibration to a plateau after activation, and the initial gelling speed can be tuned with the amount of thrombin, to have the gelling onset exactly at the desired time (this can be seen as a cross-linking method having second order kinetics versus time instead of the usual linear progression). It also means the same enzyme concentrations can be used for any macromer content, yielding always similar gelling onset time. We chose to use 20 U/ml of FXIII and 12.5 U/ml of thrombin to get the gelling onset at around 1 min, and equilibration to a plateau in 10 to 15 min.

Macromer precursor solutions of 1 to 3% gave storage moduli ranging from ∼ 0.3 to 2 kPa, which span a range of stiffness typically appropriate to encapsulate cells that are still in a proliferative and not fully differentiated state. Stable gels could still be formed down to at least 0.25% (w/v), but the high viscosity of the solutions at more than 3% make the handling of more concentrated precursors difficult. HA-TG including the MMP sensitive sequence had identical mechanical properties.

Measurement of gel diameter and swelling ratios (dry mass over wet mass) showed that the gels did not undergo any dimensional change or polymer loss compared to D0 cast gels after incubation in PBS for 4 days (Fig. 3C-D). The stable polymer content over 4 days demonstrates that essentially all HA chains were cross-linked, something which was expected for such an efficient cross-linking procedure using such a high molecular weight polymer. The lack of swelling or shrinkage, however, was a remarkable finding as it indicates a perfect balance of attraction and repulsion between the chains which allowed the gels to maintain their size at each concentration examined. Every part of the polymer chain likely play a role in the auspicious shape stability, with negative charge of the HA contributing repulsion, peptides contributing positive charges, and the spacers hydrophobicity. This is in contrast to most hydrogel systems where chain-chain and chain-solvent interactions lead to significant gel shrinkage or swelling. In these cases the unpredictable concentration- and time-dependent shape change can cause tissue engineered constructs to delaminate or pop out (dislocate, bulge) from their site of implantation.

### 2.4 Adhesion to cartilage explants

Adhesion to cartilage explants was studied with push-out tests of cylinders of gels made in 4 mm rings of bovine cartilage (Fig. 4). The adhesion was compared to fibrin, a common surgical glue and sealant and alginate, which is the most common hydrogel for 3D chondrocyte culture but is not adhesive (Kuo and Ma, Biomaterials 2001, 22, 511; Drury and Mooney, Biomaterials 2003, 24, 4337). The HA-TG was found to be significantly more adhesive to cartilage than fibrin glue, which was itself more adhesive than alginate (student t-test p<0.05). When the cartilage surface was treated with chondroitinase, to remove the polysaccharides and expose the protein part of the cartilage matrix, which is what mediates the adhesion in all three cases, the adhesion strength was found to be increased by approximately 2, 3, and 6 fold for fibrin, HA-TG, and alginate respectively. Alginate and fibrin were becoming comparable, but HA-TG was still sticking ∼3 times stronger than the controls, reaching an adhesion strength of more than 6 kPa.

### 2.5. Cartilage formation from encapsulated ECPs

Finally, hCCs were encapsulated in the hydrogels to study their potential to support chondrogenesis and effective cartilage tissue formation. The outcome was analyzed after 3 weeks in culture (D21), and alginate gels, being a standard in the field, were used as a reference.

Live/dead assays showed nearly 100% viability in all conditions (Fig. 5A), but cell morphology as seen from calcein (whole cell) and actin staining (Fig. 5B) was strongly dependent on polymer concentration. Cells spread and proliferated to fill almost the whole space in the softest gels, whereas they only formed small clusters without spreading in the strongest gels. Second harmonic generation (SHG) at 60 micron depth from the surface was collected simultaneously with calcein/PI fluorescence. This provided a way to check overall collagen production in a non-type-specific way directly on the living cells. The soft gels clearly stood out from the rest for being completely filled with a dense collagen network, whereas the other conditions just showed collagen deposition in a thin layer around the cell clusters. The collagen appeared fibrillar on the very surface of the gel, which is commonly seen from collagen 1 in such engineered cartilage constructs, but more homogeneous inside the gel, though it is deposited in the shape of the spread cells.

We then investigated the matrix deposition at gene and protein level with qPCR normalized to D0 and immunofluorescence, respectively. In all conditions, collagen 2 gene expression increased at least10⁴fold, whereas collagen 1 upregulation was less than 10 fold, indicating good induction of chondrogenesis in all conditions. The high collagen 2 expression was also translated to the protein level: the soft gels were entirely filled with a collagen 2 matrix, whereas the stiffer gels had only thin and more compact collagen 2 deposition around the cell clusters, reminiscent of what was seen in SHG. The collagen 2 / collagen 1 ratio was higher in stiffer gels, which was expected as these cells had a rounder cell morphology. Aggrecan was upregulated on the order of 10 fold in all conditions, again with higher expression in the stiffer gels. Aggrecan deposition was particularly strong on the HA gels surface.

The pattern of protein deposition was strongly depth-dependent, which can be attributed to that fact that the hydrogels were cultured in cylindrical PDMS casters bound to coverslips. The stainings clearly show better access to nutrients/growth factors/oxygen at the surface strongly benefits protein production.

Finally, the most important parameter for clinical applications, the evolution of the mechanical properties over time, was evaluated. While it was found that stiff 3% gels essentially maintain their stiffness over the 3 week culture period, soft 1 % gels showed a tremendous increase in stiffness from an initial compressive modulus of ∼1 kPa to a final value close to 0.3 MPa. Native cartilage measured in the same conditions was found to be ∼1 MPa, consistent with reported literature values. It is particularly noteworthy that the stiffness of the soft gels reached the order of magnitude of native cartilage in a clinically-relevant time frame. Intermediate 2% gels had values between these two extremes, and alginate gels were too unstable to be measured at D0 and only reached 10 kPa at D21. It is important to note that while the alginate gels underwent significant shrinkage and shape change, all of the HA-TG gels had nearly perfectly stable shape and size over the culture time. These stiffness results strongly correlate with the collagen deposition as seen by SHG that only shows assembled matrix, whereas gene expression was not the best predictor of physical outcome.

All the cell experiments were performed as well with MMP sensitive HA-TG gels, and none of the characterizations showed a significant difference between MMP sensitive and insensitive gels (data shown in supplementary). These results suggest that the increased cost and risk of premature gel degradation when using MMP sequences is not warranted, and that the use of HA-TG without MMP sequences is the best choice for cartilage tissue engineering.

Human chondroprogenitors encapsulated in the gels showed tunable proliferation and good cartilage matrix deposition, transforming the gels into cartilage-like tissue within three weeks. Strikingly, the softest 1% gels showed a tremendous increase in stiffness over the culture time, reaching a stiffness of the same order of magnitude as native cartilage.

### 2.6 Heparin-comprising HA gels

Firstly, histological results show that the burst release of TGF-b1 from HA-TG scaffolds that do not comprise heparin or heparan sulfate, is less than ideally sufficient for chondrogenesis (see Fig. 17). As can be observed in Fig. 18, the alcian blue staining is very faint and most likely due to the presence of carboxylic acid groups in the hyaluronan backbone. There is almost no collagen II and collagen I appears at the edges of the scaffold. One of the duplicates shrank.

However, when Heparin-Gln was crosslinked to the HA-TG backbone, the growth factor could efficiently promote the secretion of glycosaminoglycans and collagen II. The staining shows an even higher content of collagen II compared to the scaffolds cultured in medium containing 10 ng/ml of TGF-b1. Of great interest, the addition of heparin also led to a drastic decrease of collagen I production by the cells, indicating that there is retention of a cartilage phenotypte. Of note, the scaffold cultured in total absence of growth factor led to cell death and the gels were too soft to be further analyzed.

### 4. Experimental Section

All chemicals were purchased from Sigma-Aldrich and cell culture reagents from ThermoFisher Scientific unless stated otherwise.

### HA-SH synthesis:

400 mg (1 mmol of the disaccharide repeat unit) of HA sodium salt (Lifecore Biomedical, 1.01-1.8 MDa), and 23.8 mg (0.1 mmol) of 3,3'-dithiobis(propanoic dihydrazide) (DTPHY, Frontier Scientific) were dissolved in a 150 mM MES solution with occasional gentle swirling (final pH 4.1). Then 38.4 mg (0.2 mmol) of 1-ethyl-3-(3-dimethylaminopropyl)carbodiimide (EDC, Fluka) was dissolved in 1 ml of deionized water and added dropwise with stirring. The stirring was stopped and the reaction was allowed to continue overnight. The pH increased towards -4.5 during the course of the reaction, with MES buffer preventing it from going higher. Then, 143.33 mg (0.5 mmol) of TCEP-HCl (Fluorochem) was dissolved in 500 ml water and added, the solution was homogenized by swirling, and the reduction was left to proceed overnight in a standing sealed flask. Finally, 1 g (17 mmol) of NaCl was added to the solution, and the mixture was dialyzed against ultrapure water balanced to pH 4.5 with dilute HCl, with 4 water changes over 24h, to yield a solution of pure HA-SH sodium salt.

### HA-VS synthesis:

The HA-SH solution recovered from the previous dialysis was added dropwise into a solution of 1 ml (10 mmol) divinyl sulfone (DVS) in 40 ml of triethanolamine (TEOA) buffer, 300 mM, pH 8.0. The reaction was left to proceed for 2h at RT, then 1 g of NaCl was added and the solution was dialyzed against ultrapure water to yield pure vinyl sulfone-substituted HA (HA-VS).

The substitution rate was measured by comparing the peaks at 6.75 ppm (vinyl sulfones) and 1.75 ppm (N-acetyls from HA) from proton NMR in D₂O and found to be 10% of the carboxylic acids on HA.

HA-VS is stable for extended periods of time in solution (at least weeks), but hardly resuspends if frozen or lyophilized, probably due to hydrophobic interactions. We therefore proceeded directly with the next step (if storage in dry form is necessary, lyophilizing in the presence of >20 mM NaCl yields a powder that can be easily resuspended).

### HA-TG/Lys and HA-TG/Gln:

The HA-VS was split in two equal parts. One half was substituted with a FXIIIa substrate peptide that provides a reactive glutamine residue (TG/Gln: NQEQVSPL-ERCG (SEQ ID NO 19)) and the other half with the peptide providing the reactive lysine (TG/Lys: FKGG -ERCG (SEQ ID NO 01)). For a matrix metallo-proteinase (MMP) sensitive version of the gels, a lysine donor with an MMP-sensitive sequence (Ehrbaret al., Biomaterials, vol. 28, no. 26, pp. 3856-66, Sep. 2007) was used instead (MMP-TG/Lys: FKGG-GPQGIWGQ-ERCG (SEQ ID NO 07)). Peptides (Anawa) contained ∼40% salt which was taken into account in the molarity calculations. For conjugation, 10 ml of TEOA buffer 300 mM, pH 8.0 was added to each HA-VS portion, the solutions were deoxygenated by bubbling with nitrogen gas, and finally the peptides were added at 1.3 excess over the VS. The solutions were quickly homogenized, the flasks sealed, and the reactions left to proceed overnight without stirring. Finally, 2 g of NaCl were dissolved in each flask, and the products were dialyzed against ultrapure water. The resulting pure HA-TG components were sterilized by 0.4 µm filtration, aliquoted, and lyophilized under sterile conditions.

### HA-TG gel formation:

Aliquots of HA-TG/Lys and HA-TG/Gln were resuspended at 1, 2, or 3% (w/v) in sterile filtered TBS (NaCl 150mM, CaCl2 50 mM, TRIS 50 mM, balanced to pH 7.6). Then, the two solutions were combined in equal volume to form HA-TG polymer stock. To trigger the gelation of 60 µl of HA-TG solution, 1.5 µl of thrombin solution (Baxter, 500 U/ml) followed by 6 µl of FXIII solution (Fibrogammin, CSL Behring, 200 U/ml) were added. Gelation occurred in ∼1 min, which left enough time to transfer the liquid precursor.

Alginate gel formation: Alginate (Novamatrix) at 0.33% (w/v) (matching the stiffness of 3% HA-TG gels) in NaCl 150mM was placed in 4 mm diameter - 1 mm height cylindrical PDMS casters on coverslips, and gelled in -500 µl of 100 mM CaCl2 solution for 1 h, diffused through a pre-wetted membrane. The membrane and calcium were then removed and the gel covered with culture medium.

### Modification of Heparin

To be covalently crosslinked to the HA-TG backbone, the inventors modified Heparin (Sigma, H3393) by grafting glutamine-donor peptides (abbreviated as Gin) to the carboxylic groups of heparin, using the exact same protocol as shown above for hyaluronan. The degree of substitution was 15%.

### ELISA

For each condition study, 3 gels of 30 µl were prepared. Briefly, 50 µl of 4% HA-TG (in TBG buffer) were mixed with 50 µl of 0.2% Heparin-Gln, together with 0.1 µl of a stock solution of TGF-b1 (10 µg/ml, leading to a final amount in the gels of 1 ng). Gelation was obtained by addition of 2.5 µl of Thrombin 500U (1/40^{th}) and 5 µl of FXIII (1/20^{th}) and the gels were kept at 37°C for 30 minutes. There were then transferred to 1.5 ml Eppendorf tubes and 200 µl of supernatant (PBS, 1% BSA) was added. Between each uptake, the tubes were kept at 37°C. An ELISA kit (R&D Systems, DY240-05) was used as per manufacturer's protocol to determine the concentration of TGF-b1 in the supernatant.

### Preparation of the gels with cells and culture conditions

Human cells (375 million per 25 µl scaffold) were encapsulated in either HA-TG alone, HA-TG with TGF-b1 or HA-TG/Heparin-Gln/TGF-b1. TGF-b1 was used at a concentration of 2 µg/ml in the gels.

### • HA-TG

250 µl of 2% HA-TG in TBG buffer without calcium were gently mixed with 3.75 million cells and crosslinking was triggered by addition of 6.25 µl of Thrombin 500, 12.5 µl of FXIII and 2.5 µl of a 5M calcium chloride solution. 8 gels of 25 µl were casted in PDMS molds, of which 2 were cultured for 3 weeks in full chondrogenic media containing 10 ng/ml of TGF-b1 and 2 were cultured with control media (DMEM 31966 + 1% ITS, 40 µg/ml L-proline and 50 µg/ml ascorbic acid, no growth factor added). Media was changed every second day.

### • HA-TG with TGF-b1

30 µl of 4% HA-TG in TBG buffer without calcium, containing 100 ng of TGF-b1 (obtained by mixing 18 µl of 6.7% HA-TG with 12 µl of a stock solution of TGF-1 at 10 µg/ml), was added to 30 µl of TBG buffer without calcium. After gently mixing with 900,000 cells (to get a final cell concentration of 15 million cells/ml), gelatin was triggered by addition of 1.5 µl of Thrombin 500, 3 µl of FXIII together with 3 µl of a 1 M calcium chloride solution. 2 gels of 25 µl were produced in PDMS molds. Scaffolds were cultured in control media for 3 weeks, medium was changed every second day.

### • HA-TG/Heparin-Gln/TGF-b1

30 µl of 4% HA-TG in TBG buffer without calcium, containing 100 ng of TGF-b1 (obtained by mixing 18 µl of 6.7% HA-TG with 12 µl of a stock solution of TGF-1 at 10 µg/ml), was added to 30 µl of 0.2% Heparin-Gln in TBG buffer without calcium. After gently mixing with 900,000 hCCs (to get a final cell concentration of 15 million cells/ml), gelatin was triggered by addition of 1.5 µl of Thrombin 500, 3 µl of FXIII together with 3 µl of a 1 M calcium chloride solution. 2 gels of 25 µl were produced in PDMS molds. Scaffolds were cultured in control media for 3 weeks, medium was changed every second day.

### Histology

After 3 weeks in culture, the gels were harvested, fixed in 4% paraformaldehyde, washed in PBS and immersed in 10% sucrose (in PBS) for 1 h, followed by 30% sucrose (in PBS) overnight at 4°C. The following day, the gels were kept for 2 hours in OCT at room temperature prior to being snap frozen, by immersion in methanol containing dry ice. The constructs were cut at the cryostat (5 µm thickness) and the slides were dried and further kept at -20°C. Alcian blue staining, collagen I and II immunostainings were performed.

### Results

### Growth factor release

The covalent addition of heparin to the HA-TG hydrogel allowed the efficient retention of the growth factor in the scaffold and allowed a sustained and slow release of TGF-b1.

### Measurements

Any measurement values given herein correspond to the following methods, where applicable, unless stated otherwise

### Shear moduli measurements:

After adding the FXIII and thrombin, the gel precursor was quickly loaded onto an Anton Paar MCR 301 rheometer equipped with a 20 mm plate-plate geometry and metal floor, pre-warmed to 37°C and with humidified chamber. The probe was quickly lowered to measuring position (0.1 to 0.2 mm, monitoring the gel precursor forming a ring around the geometry while lowering the probe to ensure the measuring space is precisely filled). The gelation was then monitored at 1 Hz with 4% strain, which was within the linear viscoelastic range of the gels.

### Compressive modulus measurements:

Gels were left to swell for at least 2 days in PBS and tested under unconfined compression using a TA.XTplus Texture Analyzer (Stable Microsystems) with a 500 g load cell. The samples were compressed to a final strain of 10% at a rate of 0.01 mm/s. The compressive modulus E reported is the slope of the initial linear range of the stress-strain curve.

### Adhesion strength measurements:

Bovine articular cartilage samples with 1-2 mm thickness were harvested from the knee joints of calves aged 3-6 months. Cartilage rings of 8 mm in outer diameter and 4 mm inner diameter were prepared using biopsy punches and washed in PBS. The explants were then randomly divided in two groups: one group was left in PBS while the other was incubated for 15 minutes at 37°C in 1 U/ml chondroitinase ABC followed by 3 washes with PBS. The chondroitinase digestion results in approximately 50 µm digestion of GAGs as confirmed by Alcian blue staining . HA-TG gels were injected into the circular hold of the cartilage explants and left to gel for 20 minutes at 37°C in a humidified chamber. To prevent leakage the explants were laid on a parafilm-coated surface. Push-out tests were performed at 0.5 mm/s rate with a 3 mm rod. The bond strength was calculated as the maximum force divided by the area of the inner punched hole.

*hCCs isolation and expansion protocol:* hCCs were harvested and isolated as described by Darwiche et al. (Darwiche 2012). Briefly, a tissue biopsy from the proximal ulnar epiphysis of a 14-week gestation donor was taken and minced. hCCs grew out of the tissue pieces. Cells were cultured for up to 2 weeks in DMEM (cat. 41966) containing 10% v/v FBS, 2mM L-glutamine, and 10 µg/ml Gentamycin. The cells were stored in liquid nitrogen, and expanded to passage 3 before encapsulation.

*hCC encapsulation in gels:* Cells were trypsinized and resuspended at 15x10⁶ cells/ml in the HA-TG solution, then the gelation was triggered as described previously. The gels were quickly cast in 4 mm diameter UV-sterilized PDMS cylindrical molds (SYLGARD 184, Corning) adhered to 10 mm coverslips. The gels were allowed to crosslink for 15 minutes at 37°C before adding chondrogenic medium (DMEM (cat. 31966) supplemented with 10 ng/ml transforming growth factor β3 (TGF-β3, Peprotech), 100 nM dexamethasone, 50 µg/ml L-ascorbate-2-phosphate, 40 µg/ml proline, 0.5% penicillin-streptomycin, and 1% ITS+ Premix (Corning)). The gels were incubated in a controlled humidified chamber (37°C, 5% (vol/vol) CO₂) for 3 weeks and the culture media was replaced twice a week.

*Live imaging:* Gels were incubated for 1h in medium supplemented with 2 µM calcein AM and 6.6 µg/ml propidium iodide (PI), washed with fresh medium, and imaged on a Leica SP8 multiphoton microscope (25x water immersion objective, Mai Tai irradiation at 900 nm) with simultaneous collection of second harmonic generation (SHG) and fluorescence. The pictures presented were taken 60 to 70 µm from the sample surface, as the signal deteriorated at deeper depths.

*Immunohistochemistry:* Gels were fixed in 4% formaldehyde for 45 minutes, embedded in O.C.T (Tissue-Tek O.C.T Compound Blue, Sysmex) and stored at -80 °C. 5 µm thick sections were cut using a Cryostat (CryoStar NX70, Thermo Scientific). Collagen 1 and 2 staining were performed after 30 minutes of 0.2% (w/v) hyaluronidase digestion at 37 °C and 1 hour blocking with 5% BSA in PBS with 1:200 diluted mouse anti-collagen 1 (Abcam #ab6308) and 1:200 diluted rabbit anti-collagen 2 (Rockland 600-401-104). Proteoglycan staining was performed after reducing the tissue with 10mM dithiothreitol in TBS pH 7.4 for 2 hours at 37°C and alkylating with 40 mM iodoacetamide in PBS for 2 hours at 37°C. Sections were then digested with 0.02 U/ml chondroitinase ABC for 40 minutes at 37°C and blocked for 1 hour at room temperature with 5% BSA before incubating with primary antibody (Hybridoma 12/21/1-C-6). All primary antibodies were diluted in 1% (w/v) BSA in PBS and incubated overnight at 4°C. Alexa Fluor 594 Goat Anti-Mouse IgG (Invitrogen, A11005), Alexa Fluor 488 Goat anti-rabbit Alexa 488 (Invitrogen A11008) and Alexa Fluor 488 Goat anti-mouse (Invitrogen A11029) secondary antibodies were used at 1:200 dilution in 1 % BSA in PBS for 1 hour at RT. Finally, slides were incubated for 10 minutes with the nuclear stain DAPI (Molecular Probes, Invitrogen) before mounting with VectaMount AQ Mounting Medium (Vector Laboratories).

*RNA extraction and PCR:* Samples were frozen in liquid nitrogen and crushed using pellet pestles (Thomas Scientific). Total RNA was prepared using NucleoSpin miRNA kit (Macherey-Nagel) and concentration was determined with a microplate reader Synergy H1 (BioTek Instruments). RNA with an absorbance ratio at 260/280 nm between 1.9 and 2.1 was used for PCR analysis. The Fast SYBR Green Master Mix (Applied Biosystems) was used to perform the PCR amplification with 150 nM forward and reverse primer. All primers (see Table 1) were designed across exon-exon junctions using Real Time PCR Design Tool from Integrated DNA Technologies (http://eu.idtdna.com/Scitools/Applications/RealTimePCR/Default.asp) to avoid the amplification of genomic DNA. All data came from 3 independent replicates and was analyzed using the 2-ΔΔCt method) and normalized against the reference gene RPL13a (Studer et al. Tissue Eng Part C Methods. 2012 Oct;18(10):761-71) with day 0 samples chosen as reference.

| mRNA | Accession n° | BP | | Primer sequence (5'-3') |
|---|---|---|---|---|
| *hRPL13a* | NMR_012423 | 100 | FWD | AAGTACCAGGCAGTGACAG (SEQ ID NO 030) |
| | | | REV | CCTGTTTCCGTAGCCTCATG (SEQ ID NO 031) |
| *hCol1a1* | NMR_000088 | 83 | FWD | CAGCCGCTTCACCTACAGC (SEQ ID NO 032) |
| | | | REV | TTTTGTATTCAATCACTGTCGCC (SEQ ID NO 033) |
| *hCol2a1* | NMR_001844 | 92 | FWD | GGAATTCGGTGTGGACATAGG (SEQ ID NO 034) |
| | | | REV | ACTTGGGTCCTTTGGGTTTG (SEQ ID NO 035) |
| *hCol10a1* | NMR_000493 | 108 | FWD | ATTCCTAGTGGCTCCAATGTG (SEQ ID NO 036) |
| | | | REV | GCCTACCTCCATATGCATTTT (SEQ ID NO 037) |
| *hACAN* | NM_001135.3 | 98 | FWD | GAATGGGAACCAGCCTATACC (SEQ ID NO 038) |
| | | | REV | TCTGTACTTTCCTCTGTTGCTG (SEQ ID NO 039) |

### Example 2: Hyaluronan hydrogels supporting the in vitro formation of 3D neuronal networks

### Introduction

Hyaluronan is the backbone of the extracellular matrix of the brain, where it has multiple structural and signalling roles. Long hyaluronan chains (>1 MDa) are bound by chondroitin sulfate proteoglycans such as aggrecan which are in turn cross-linked by tenascins. The brain doesn't have an interpenetrating collagen 2 network, which makes hyaluronan an even more essential component of the brain matrix, and the most obvious choice to mimic the natural extracellular matrix of neurons.

Neuron are among the most fragile cells in the body, and using a cross-linking chemistry that is absolutely free of stressful reactive chemicals is therefore a strong advantage when encapsulating them into a hydrogel, in order to preserve high viability. For this, FXIIIa cross-linking is particularly adapted: the enzyme selectively cross-links the peptides bound to HA-TG, and none of the gel components can cross-react with the cells.

Additionally, neurons thrive in very soft matrices (∼100 Pa) and the most natural way to achieve such low stiffness in a stable and reliable way is to cross-link high molecular weight polymers with a low cross-linking density. For these reasons, the high molecular weight hyaluronan with low transglutaminase substrate peptide substitution described for the cartilage example (HA-TG) is perfectly suited. The gels can be made to match the right stiffness for neural tissue by reducing the HA-TG percentage to ∼0.5% (w/v), without sacrificing on the stability.

### Results and discussion

### Neuron encapsulation: viability and morphology

As seen on Figure 9A, gelling can be adjusted to happen within the first minute after sample mixing and delivery, and stiffness from 10 to 2000 Pa is easily accessible with variations of the HA-TG concentration. Pilot experiments of neuron encapsulation found that 0.25% gels tended to be too soft to be easily handled without damage, whereas 1% gels had reduced neurite outgrowth. We therefore conducted the 3D neuron culture study with 0.5% HA-TG (half of it being HA-TG/Lys, and half of it HA-TG/Gln). These gels did not appear to degrade, nor did they swell or shrink noticeably over culture periods of typically 1 to 2 months.

Being inert in the absence of FXIIIa had the expected consequence that HA-TG reagents had no cytotoxicity whatsoever, and dissociated neurons could be encapsulated without loss of viability. In 0.5% (w/v) gels, the viability was around 90% just before encapsulation (D0), and found to be of 85±5% at D2 and 81±7% at D5 (SD n=9). The gels showed no sign of degradation at any time point and stable cultures could be kept for more than 2 months.

Fast neurite extension was seen from the first days, and by D21, the gels were filled by a dense mesh of neurites, forming extensive 3D networks (note the reduced MIP thickness and increased neurite densities in Fig. 12A from D2 to D21). Large growth cones with many exploratory actin filled filipodia at the tip of microtubule-filled axons, as well as smaller and simpler dendritic growth cones, were reminiscent of what is seen *in vivo* (Fig. 12B close-ups a-b). Neurites were also covered with many actin-filled small filaments at D2, that might be branching points initiations. By D5, they appeared mostly smooth. Afterwards, actin-filled buds reminiscent of dendritic spines started to be visible on some neurites at D5, and became omnipresent by D21 (clearly visible in Fig. 12A D2-D21 and Fig. 12B close-ups b-c-d).

Specific axonal and dendritic markers appeared at the same time as the morphological characteristics associated with axons and dendrites: Tau1 positive axons were already visible at D2, and the dendritic marker MAP-2 started to be expressed in the cell bodies and proximal dendrites at D5, and became strongly expressed and localized to the whole length of a subset of neurites by D21 (Fig. 13). βIII tubulin stains all neurites from these embryonic neurons, as expected, while neurofilaments were strongly expressed only after 21 days, and only in a subset of neurites.

### Spiking activity

The genetically encoded calcium reporter GCaMP was used to monitor spiking activity of the neurons. Strong and fully synchronous spiking was already visible at D10 (Fig. 14E-F). In agreement with this, a great density of synaptotagmin-filled potential presynaptic terminals and PSD-95 filled potential post-synaptic terminals were found associated with cell bodies and neurites (Fig. 14A-B) and in many cases associated with each other (C-D), to form synapses.

As calcium waves can be present in other cell types than neurons and have other causes than electrical spiking, we confirmed the spiking was indeed neuronal electrical activity by blocking with the specific voltage gated sodium channel blocker Tetrodotoxin (TTX). In addition, specific inhibitors of glutamatergic transmission CPP and CNQX blocked the spiking activity as well, proving the neuronal excitation is based on standard glutamatergic excitation, as could be expected from cortical neurons (Fig. 15). Activity was always recovered after washing overnight, showing none of the compounds were used at a toxic dose.

The ease with which neurons in 3D culture in these gels can be monitored with fluorescent reporters together with the fact that pharmacological testing with small molecules can be done by simply letting molecules diffuse 1h through the gels makes these gels particularly fitting for neurobiology or pharmacology studies in 3D cultures.

An additional note should be made that these HA-TG gels were also found to adhere to mouse spinal cord tissue (Fig 9C), which is an important property for in vivo translation of our in vitro results: for example to fill a cyst in the spinal cord with an axonal permissive matrix, or to deliver cells in the brain with a supportive matrix.

### Conclusion

The new HA-TG hydrogels described here cross-link with the specific transglutaminase activity of FXIIIa and present an ideal set of properties for neural tissue engineering, including chemical stability, specific cross-linking with independently tunable gelation speed and stiffness, cytocompatibility with dissociated neuron encapsulation (the most demanding cytocompatibility test), injectability, covalent cross-linking to fibrin and other proteins, and the possibility to enzymatically degrade the gels for bioresorption or cell recovery. These gels are additionally based on high MW HA, which has important neuroprotective, anti-fibrotic, and anti-inflammatory properties. HA is additionally the backbone of the brain ECM, so neurons are provided with a gel which mimics their natural environment. All these properties enabled 3D neuronal cultures of unprecedented performance, with fast neurite outgrowth, proper neuron polarization, quickly-occurring and long-lasting electrical activity with strong synaptic connectivity. These gels are therefore perfectly positioned to bring neurobiological and pharmacologic cell-based studies to the third dimension. They are promising as well for translation to brain or spinal cord injury applications, something which will require further *in vivo* investigation.

### Materials and methods

All reagents were used at highest purity available unless stated otherwise.

### HA-SH synthesis, HA-VS synthesis, peptide conjugation:

See the experimental section of Example 1.

### FXIII pre-activation

FXIII (Fibrogammin, CSL Behring) was resuspended at 200 U/ml in water, aliquoted by 100 ul and stored at -80°C. For activation, 3.2 µl of thrombin (from Tissucol, Baxter) at 50 U/ml and 2 µl of 100 mM CaCl2 were added to one aliquot, and incubated for 15 min at 37°C. Activated FXIII (FXIIIa) was stored as 10 µl aliquots at -80°C until the day of use.

### HA-TG gel formation

Aliquots of HA-TG/Lys and HA-TG/Gln were resuspended at the desired concentration, i.e. 0.5% (w/v) unless indicated otherwise, in saline supplemented with 50 mM TRIS and 50 mM CaCl2, with pH balanced to 7.6 and sterilized by filtration (referred to as TBS). Then, the two solutions were combined in equal volume, and the amount of enzyme corresponding to the desired gelling speed was added to the wall of the tube, i.e. 3 µl of FXIIIa 200 U/ml for 80 µl of gel unless indicated otherwise, and incorporated quickly by vortexing. Gelation occurred within 1-2 minutes, and was left to proceed for 20 minutes to ensure a stiffness plateau is reached before transferring to medium.

### Rheometry

After adding the FXIIIa, the gel precursor was quickly loaded on an Anton Paar MCR 301 rheometer equipped with a 20 mm plate-plate geometry and metal floor, pre-warmed to 37°C and with humidified chamber. The probe was quickly lowered to measuring position (100 to 200 µm, monitoring the gel precursor forming a crown around the geometry while lowering the probe to ensure the measuring space is precisely filled). Then the gelling was monitored at 1 Hz with 4% strain.

Control fibrin gels were made by adding FXIIIa and thrombin at 7.5 and 0.5 U/ml respectively from concentrated stocks into a 0.5% (w/v) fibrinogen solution in TBS. HA-Fibrin hybrids were made by adding the same amount of enzyme in a fibrinogen solution additionally containing 0.5% (w/v) HA-TG.

For measurements after swelling, 0.5% (w/v) HA-TG gels were made in 1.4 mm thick Teflon casters, left to gel for 20 min at 37°C with a humidified atmosphere, collected and submerged in PBS pH7.4 for 3 days. The gels were then loaded on the rheometer equipped with a 10 mm plate-plate geometry, compressed by 10% to ensure surface adhesion, trimmed, and measured with a frequency sweep at 4% strain.

### Neuron cultures

Cortices from E17 Wistar rat embryos were dissected and dissociated as described previously. Briefly, the cortices were incubated for 15 min at 37°C in PBS supplemented with 1 mg/ml BSA, 10 mM glucose, 0.5 µg/ml DNAse (Sigma, D-5025) and 0.5 mg/ml papain (Sigma, P-4762), and washed with blocking medium consisting of DMEM + 10% FBS. They were resuspended in 2 ml of blocking medium, and dissociated by trituration using a fire polished Pasteur pipette. The dissociated cells were resuspended in serum free growth medium, consisting of Neurobasal + B27 (Gibco 21103-049 and 17504-044) supplemented with 1x Glutamax and Penicillin/Streptomycin, and plated overnight on poly-L-lysine coated flasks (50 ug/ml in borate buffer, pH8.4, incubated at 37°C for 1h). The following day, the plated neurons were first washed with TrypLE express for 5 min, to detach weakly adherent non-viable cells and cell debris. The remaining healthy cells were then detached with trypsin/EDTA (Gibco 12605-010 and 25200-072). After addition of two volumes of blocking medium, the cells were centrifuged and resuspended in serum-free growth medium, and counted.

For encapsulation, the neurons were pelleted and resuspended at 10e6 cells/ml in the HA-TG/Lys-Gln mix. After addition of the FXIIIa, the gel precursor was cast into home-made 4 mm diameter / 1 mm height PDMS molds pre-adhered onto coverslips, and kept in 24 well plates. After 20 minutes of gelling at 37°C in a humidified atmosphere, 1 ml of serum-free medium was added onto the gels. After 2-3 weeks, half of the medium was replaced once a week.

### Live-dead assays

Gels were incubated 1h in medium supplemented with calcein AM 2 µM and propidium iodide 6.6 µg/ml, transferred to fresh medium for washing and imaged on a Leica SP8 multiphoton over 465x465x200 µm. Live and dead cells were counted manually on the maximum intensity projection (MIP) (typically ∼400 cells/image). Experiments were reproduced in triplicate with 3 different litters (n=9).

### Immunocytochemistry and imaging

Primary antibodies were: βIII-tubulin (Sigma T5076, 1:500), neurofilament (Sigma N4142, 1:150), synaptotagmin (DSHB, 8 µg/ml), MAP-2 (Sigma M3696, 1:200), PSD-95 (Abcam ab18258, 1:400). Secondary antibodies conjugated with Alexa 488 and 594 (Invitrogen A11005, A11008, A10680, A11015) were used at 1:200.

We adapted standard immunocytochemistry protocols to stain and image directly the entire gels. Samples were fixed and permeabilized for 1h at 4°C in 10% formalin with 0.1 % Triton X-100, blocked overnight with 5% BSA in PBS, and washed with PBS (twice 1h, once overnight). They were then incubated in primary antibody (overnight at RT with gentle shaking, dilution with 3% BSA in PBS), washed with PBS, incubated overnight in secondary antibody, washed with PBS, stained with DAPI 0.3 µM in PBS for 1 h, and finally washed with PBS.

Imaging of immunostained samples was performed on a Leica SP8 multiphoton microscope with a 20x/0.95 NA water objective, typically exciting at 710 nm and 1100 nm using MaiTai Deepsee and Insight Deepsee fs-lasers respectively (Spectra Physics). The resulting stacks were edited in Fiji to apply a MIP, and adjust the color display (brightness/contrast/gamma). When salt and pepper noise was present, median filtering over < 2 px was applied.

### Spiking activity imaging

The genetically encoded intracellular calcium reporter GCaMP was delivered with an adeno-associated virus added directly into the gel precursor at optimized concentration. The spiking activity was then imaged either on a Leica SP8 confocal microscope at 8 frames/s (Fig. 14) or on a Zeiss observer widefield microscope with CO2 incubation at 2.5 frames/s (Fig. 15).

To confirm that calcium waves were indeed associated with neuronal electrical spikes and glutamatergic transmission, the following selective inhibitors were added in the medium and left to diffuse into the gels for 1h: Tetrodotoxin 500 nM (Acros Organics, voltage gated sodium channel antagonist), CPP 10 µM (Sigma, NMDA glutamate receptor antagonist), CNQX 20 µM (Sigma, AMPA/kainate glutamate receptor antagonist).

### Example 3: Decellularized cartilage particles in an in-situ crosslinkable hydrogel

In this Example we present a scaffold with bioactive potential for cartilage repair consisting of an enzymatically crosslinkable modified hyaluronan (HA) hydrogel and decellularized cartilage particles (DCC). Particles decellularized with a lab own protocol and commercial BioCartilage® particles (Arthrex, Naples, FL, USA) were loaded with a chondrogenic growth factor and mixed with HA to form stable, injectable, in-situ crosslinkable hydrogels (HA-DCC). When seeded with human chondroprogenitor cells (hCCs), gels stimulated cell proliferation and synthesis of cartilaginous matrix to a higher degree compared to HA-gels without particles when cultured in presence of a growth factor.

**METHODS:** Bovine articular cartilage was harvested from condyles of calves, croymilled and decellularized with a with a two-step protocol. DCC particles and BioCartilage® were loaded with transforming growth factor beta-3 (TGFβ-3) and were mixed with a 1% (w/v) HA-TG hydrogel as shown in Examples 1 or 2 together with 15 Mio/ml hCCs. Factor XIII was added as shown in Example 1 and 2, and after activation with thrombin and Ca²⁺ crosslinking was initiated. The gels were cultured in media either with or without TGFβ-3 (10ng/mL) for 3 weeks for biochemical assays, histological analysis and mechanical testing. Culturing media was analysed with ELISA to determine TGFβ-3 release.

**RESULTS:** After culturing, HA-DCC exhibited a significantly higher elastic modulus measured in compression tests than HA gels without particles. The gels increased respectively from 1.04 and 1.61 kPa to 126 and 45 kPa (*Fig.16*) due to matrix deposition.

Cell proliferation was investigated by a Picogreen assay. All HA-DCC gels showed up to 5-fold increases of DNA after 3 weeks, whereas gels without particles showed up to a 3-fold increase in DNA.

Histological analysis confirmed deposition of GAGs and collagen II for gels cultured in presence of TGFβ-3 (media or loaded DCC). No difference was visible between bovine DCC particles and BioCartilage®.

The invention is further specified in detail in the following items:
Item 1: A process for forming a hyaluronan hydrogel, comprising the steps of
   a. providing an aqueous solution, said aqueous solution comprising
      i. a first hyaluronan peptide conjugate comprising transglutaminase donor peptides and
      ii. a second hyaluronan peptide conjugate comprising transglutaminase acceptor peptides,
      wherein said first hyaluronan peptide conjugate and said second hyaluronan peptide conjugate are each represented by a general formula I, 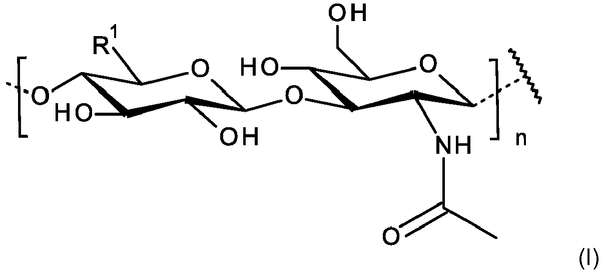 wherein 5% to 20%, particularly 8-12%, more particularly approximately 10% of R¹ moieties are represented by a general formula II*,* 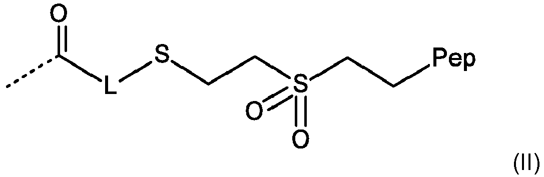 wherein
      - L is a linker moiety, particularly a linker consisting of 2, 3, 4, 5 or 6 C, N and/or O atoms in the linking chain, more particularly L is NH-Alk or O-Alk or NH-NH-CO-Alk with Alk being a C1 to C5 alkyl, even more particularly L is -N-NHCO-(CH₂)₂-, and
      - Pep is a transglutaminase donor peptide or a transglutaminase acceptor peptide, respectively, and the rest of R¹ moieties are represented by - COOH.
b. adding a transglutaminase capable of covalently linking said transglutaminase donor peptides to transglutaminase acceptor peptides, particularly
   i. a factor XIII polypeptide and a thrombin polypeptide, or
   ii. a factor XIIIa polypeptide,
   to said aqueous solution.
Item 2: A process for forming a hyaluronan hydrogel, comprising the steps of
   a. providing an aqueous solution of a first hyaluronan peptide conjugate comprising transglutaminase donor peptides and a second hyaluronan peptide conjugate comprising transglutaminase acceptor peptides,
   b. adding a thrombin polypeptide to said aqueous solution and allowing equilibration of the resulting mixture;
   c. subsequently, adding a factor XIII polypeptide.
Item 3: The process according to items 1 or 2, wherein the concentration of
   a. said thrombin polypeptide is 0.1 to 100 U/ml, particularly 1 to 20 U/ml, more particularly 12.5 U/ml; and
   b. said factor XIII polypeptide is 1 to 50 U/ml, particularly 5 to 40 U/ml, more particularly 15 to 25 U/ml.
Item 4: The process according to any one of the preceding items, wherein the aqueous solution of step a. additionally comprises heparin or heparin sulfate, particularly at a concentration from 0,05% to 0,5% (w/v relative to the gel).
Item 5: The process according to item 4, wherein the heparin or heparan sulfate comprises covalently attached transglutaminase donor and / or acceptor peptides, particularly wherein 10% to 20% of carboxylic acid groups present in said heparin or heparan sulfate are covalently modified, more particularly covalently modified to contain a modification described by general formula (II) as laid out above, with L, S and Pep having the meaning indicated above.
Item 6: The process according to any one of items 3 or 5, wherein the molecular mass of said heparin or heparan sulfate ranges from 1000 g/ mol to 50000 g / mol.
Item 7: The process according to any one of the preceding items, wherein the concentration of the sum of said first hyaluronan peptide conjugate and said second hyaluronan peptide conjugate is 0,25% (w/v) to 5% (w/v), particularly 0,75% to 0,95% or from 0, 5% to 3%, 0,5% to 2%, or 0,5% to 1,5%.
Item 8: A process for modification of a hyaluronan polymer, wherein said hyaluronan polymer is composed of n dimers of D-glucuronic acid moieties and D-N-acetylglucosamine moieties, and wherein said D-glucuronic acid moieties bear reactive carboxylic acid moieties, and said process comprises the steps of:
   a. thiolation of 5% to 20%, particularly 8-12%, more particularly approximately 10% of said reactive carboxylic acid moieties to yield partially thiolated hyaluronan;
   b. reacting said partially thiolated hyaluronan with divinylsulfone to yield vinylsulfone-hyaluronan;
   c. reacting said vinylsulfone-hyaluronan with a peptide comprising a cysteine moiety, wherein said peptide comprises a sequence selected from a transglutaminase donor peptide sequence and a transglutaminase acceptor peptide sequence.
Item 9: The process according to item 8, wherein thiolation is effected by:
   a. reacting said hyaluronan polymer with 3,3'-dithiobis(propanoic dihydrazide), particularly in the presence of an alkylcarbodiimide; followed by
   b. reacting the product of step a particularly without further workup with a reducing agent selected from TCEP (tris-2-(carboxyethyl)phosphine), DTT (dithiothreitol) and beta-mercaptoethanol, to yield the partially thiolated hyaluronan.
Item 10: The process according to any one of the preceding items, wherein said hyaluronan and/or said hyaluronan peptide conjugate and/or said second hyaluronan peptide conjugate are characterized by a mean molecular weight equal or greater than (≥) 250 kg/mol, ≥ 500 kg/mol, particularly greater than 1000 kg/mol (1 MDa); more particularly between 1000kg/mol and 4000 kg/mol, even more particularly between 1000 kg/mol and 2000 kg/mol.
Item 11: The process according to any one of the preceding items, wherein n is ≥ 2500.
Item 12: A process for modification of a heparin or heparan sulfate polymer, wherein said heparin or heparan sulfate polymer comprises D-glucuronic acid moieties and L-iduronic acid moieties, each of which bearing reactive carboxylic acid moieties, and said process comprises the steps of:
   a. thiolation of 5% to 20%, particularly 8-12%, more particularly approximately 10% of said reactive carboxylic acid moieties to yield partially thiolated heparin or heparan sulfate;
   b. reacting said partially thiolated hyaluronan with divinylsulfone to yield vinylsulfone-heparin or vinylsulfone-heparan sulfate;
   c. reacting said vinylsulfone-heparin or -heparan sulfate with a peptide comprising a cysteine moiety, wherein said peptide comprises a sequence selected from a transglutaminase donor peptide sequence and a transglutaminase acceptor peptide sequence.
Item 13: The process according to item 12, wherein thiolation is effected by:
   a. reacting said heparin or heparan sulfate polymer with 3,3'-dithiobis(propanoic dihydrazide), particularly in the presence of an alkylcarbodiimide; followed by
   b. reacting the product of step a. (particularly without further workup) with a reducing agent selected from TCEP (tris-2-(carboxyethyl)phosphine), DTT (dithiothreitol) and beta-mercaptoethanol, to yield the partially thiolated heparin or heparan sulfate.
Item 14: The process according to any one of the preceding items, wherein
   a. the transglutaminase donor peptide sequence is or comprises a sequence selected from SEQ ID NO 01 to SEQ ID 14;
   b. the transglutaminase acceptor peptide sequence is or comprises a sequence selected from SEQ ID NO 15 to SEQ ID 29.
Item 15: A composition comprising / essentially consisting of a hyaluronan polymer of general formula I, 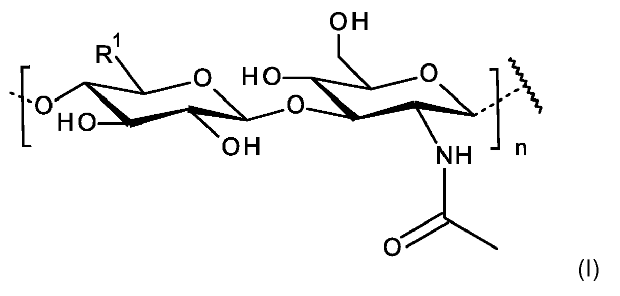 wherein 5% to 20%, particularly 8-12%, more particularly approximately 10% of R¹ moieties are represented by a general formula II, 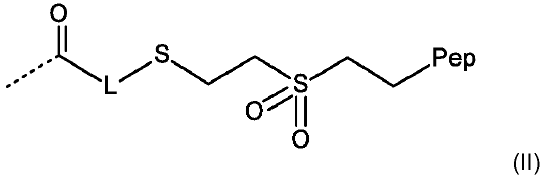 wherein
   - L is a linker moiety, particularly a linker consisting of 2, 3, 4, 5 or 6 C, N and/or O atoms in the linking chain, more particularly L is NH-Alk or O-Alk or NH-NH-CO-Alk with Alk being a C1 to C5 alkyl, even more particularly L is -N-NHCO-(CH₂)₂-, and
   - Pep is a transglutaminase donor peptide or a transglutaminase acceptor peptide, respectively, and the rest of R¹ moieties are represented by COOH.
Item 16: The composition according to item 15, wherein said hyaluronan polymer has a mean molecular weight equal or greater than (≥) 250 kg/mol, ≥ 500 kg/mol, particularly greater than 1000 kg/mol (1MDa); more particularly between 10E6g/mol and 4E6 g/mol, even more particularly between 10E6g/mol and 2*10E6 g/mol l.
Item 17: The composition according to anyone of the preceding items 15 or 16, wherein n is ≥ 2500.
Item 18: The composition according to any one of items 15 to 17, further comprising a sulfated polysugar, particularly a sulfated polysugar selected from alginate sulfate, hyaluronan sulfate, chondroitin sulfate, fucan sulfate, carrageenan, ulvan, heparin and heparan sulfate, particularly at a ratio of 2,5% to 15% (m/m) relative to the mass of hyaluronan polymer, more particularly further comprising heparin and heparan sulfate at a ratio of 2,5% to 15% (m/m) relative to the mass of hyaluronan polymer.
Item 19: The composition of item18, wherein the heparin or 44eparin sulfate comprises covalently attached transglutaminase donor and / or acceptor peptides, particularly wherein 10% to 20% of carboxylic acid groups present in said heparin or 44eparin sulfate are covalently modified, more particularly covalently modified to contain a modification described by general formula (II) as laid out above [wherein the carbon shown on the left is the carboxylic carbon comprised within the glucosaminoglycan backbone], with L, S and Pep having the meaning indicated above.
Item 20: The composition according to any one of the preceding items 15 to 19, wherein
   a. the transglutaminase donor peptide sequence is or comprises a sequence selected from SEQ ID NO 01 to SEQ ID 14;
   b. the transglutaminase acceptor peptide sequence is or comprises a sequence selected from SEQ ID NO 15 to SEQ ID 29.
Item 21: The composition according to any one of items 15 to 20, wherein the composition comprises
   - a hyaluronan polymer comprising transglutaminase donor peptides and
   - a hyaluronan polymer comprising transglutaminase donor peptides, and
   - a Factor XIII polypeptide and / or a thrombin polypeptide.
Item 22: The composition according to item 21, characterized in that the composition is provided in dried form.
Item 23: The composition according to any one of items 15 to 22, characterized in that the composition comprises a thrombin polypeptide but no factor XIII polypeptide.
Item 24: The composition according to any one of items 15 to 23, wherein the composition comprises 500 U to 2500 U thrombin per gram of hyaluronan polymer, particularly 1000 U to 1500 U per gram hyaluronan polymer.
Item 25: A hyaluronan hydrogel comprising transglutaminase cross-linked hyaluronan and water, obtained
   - by a process according to any one of items 1 to 7, or
   - by crosslinking a hyaluronan obtained by a process according to any one of items 8 to 14, or
   - by crosslinking a composition according to any one of items 15 to 24 .
Item 26: The hyaluronan hydrogel according to item 25, wherein the hydrogel comprises 0,25% to 0,99% of cross-linked hyaluronan in water (w/v), particularly 0,25% to 0,75% (w/v), or 0,4% to 0,6% (w/v), particularly ca. 0, 5% (w/v).
Item 27: The hyaluronan hydrogel according to any one of items 25 or 26, wherein the hydrogel comprises 0,5% to 4% of cross-linked hyaluronan in water (w/v), particularly 1% to 2% (w/v).
Item 28: The hyaluronan hydrogel according to any one of items 25 to 27, wherein the hydrogel comprises a growth factor selected from transforming growth factor beta 1, 2, or 3, insulin like growth factor 1 and fibroblast growth factors 1, 9, or 18.
Item 29: The hyaluronan hydrogel according to item 28, wherein the concentration of said growth factor is between 10-2500 ng/mL.
Item 30: The hyaluronan hydrogel according to any one of items 25 to 29, characterized by a pore size of 0.1 µm to 1.0 µm, particularly 0.2 µm to 0.8 µm.
Item 31: The hyaluronan hydrogel according to any one of items 25 to 30, wherein said hydrogel is characterized by an adhesion strength, as measured by push-out assay,
   a. of more than 0.5 kPa, in particular 1 to 10 kPa, more particularly around 2 kPa when adhesion to a cartilage surface is measured; and / or
   b. of more than 4 kPa, particularly approx. 6 kPa when adhesion to a chondroitinase treated cartilage surface is measured.
Item 32: The hyaluronan hydrogel according to any one of items 25 to 31, characterized by a change in dimension (swelling or shrinking), as measured by comparing gel disk diameters after gelation and after additional swelling in an isotonic aqueous buffer for 3 days, of less than 20% on average, in particular less than 10% on average, more particularly less than 4%.
Item 33: The hyaluronan hydrogel according to any one of items 25 to 32, additionally comprising chondrogenic cells, particularly chondrocytes, chondroprogenitors, mesenchymal stem cells, adipose stem cells.
Item 34: The hyaluronan hydrogel according to any one of items 25 to 32, wherein said hydrogel comprises neurons, neural cells and/or neuroprogenitor cells.
Item 35: The hyaluronan hydrogel according to any one of items 25 to 34, further comprising particles and/or fibres derived from cartilage.

## Claims

1. A process for forming a hyaluronan hydrogel, comprising the steps of
a. providing an aqueous solution, said aqueous solution comprising
i. a first hyaluronan peptide conjugate comprising transglutaminase donor peptides and
ii. a second hyaluronan peptide conjugate comprising transglutaminase acceptor peptides,
wherein said first hyaluronan peptide conjugate and said second hyaluronan peptide conjugate are each represented by a general formula I, 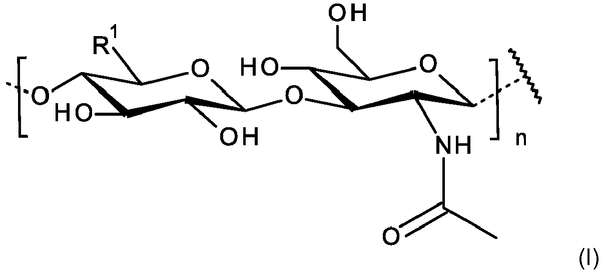 wherein 5% to 20%, particularly 8-12%, more particularly approximately 10% of R¹ moieties are represented by a general formula **II,** 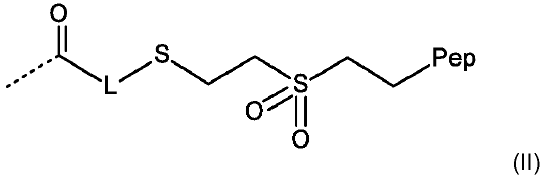 wherein
- L is a linker moiety, particularly a linker consisting of 2, 3, 4, 5 or 6 C, N and/or O atoms in the linking chain, more particularly L is NH-Alk or O-Alk or NH-NH-CO-Alk with Alk being a C1 to C5 alkyl, even more particularly L is -N-NHCO-(CH₂)₂-, and
- Pep is a transglutaminase donor peptide or a transglutaminase acceptor peptide, respectively, and the rest of R¹ moieties are represented by -COOH.
b. adding a transglutaminase capable of covalently linking said transglutaminase donor peptides to transglutaminase acceptor peptides, particularly
i. a factor XIII polypeptide and a thrombin polypeptide, or
ii. a factor XIIIa polypeptide,
to said aqueous solution.

2. A process for forming a hyaluronan hydrogel, comprising the steps of
a. providing an aqueous solution of a first hyaluronan peptide conjugate comprising transglutaminase donor peptides and a second hyaluronan peptide conjugate comprising transglutaminase acceptor peptides,
b. adding a thrombin polypeptide to said aqueous solution and allowing equilibration of the resulting mixture;
c. subsequently, adding a factor XIII polypeptide.

3. The process according to any one of the preceding claims,
wherein the aqueous solution of step a. additionally comprises heparin or heparan sulfate, particularly at a concentration from 0,05% to 0,5% (w/v relative to the gel), and
wherein the heparin or heparan sulfate comprises covalently attached transglutaminase donor and / or acceptor peptides, particularly wherein 10% to 20% of carboxylic acid groups present in said heparin or heparan sulfate are covalently modified, more particularly covalently modified to contain a modification described by general formula (II) as laid out above, with L, S and Pep having the meaning indicated above.

4. The process according to any one of the preceding claims, wherein the concentration of the sum of said first hyaluronan peptide conjugate and said second hyaluronan peptide conjugate is 0,25% (w/v) to 5% (w/v), particularly 0,75% to 0,95% or from 0,5% to 3%, 0,5% to 2%, or 0,5% to 1,5%.

5. A process for modification of a hyaluronan polymer, wherein said hyaluronan polymer is composed of n dimers of D-glucuronic acid moieties and D-N-acetylglucosamine moieties, and wherein said D-glucuronic acid moieties bear reactive carboxylic acid moieties, and said process comprises the steps of:
a. thiolation of 5% to 20%, particularly 8-12%, more particularly approximately 10% of said reactive carboxylic acid moieties to yield partially thiolated hyaluronan;
b. reacting said partially thiolated hyaluronan with divinylsulfone to yield vinylsulfone-hyaluronan;
c. reacting said vinylsulfone-hyaluronan with a peptide comprising a cysteine moiety, wherein said peptide comprises a sequence selected from a transglutaminase donor peptide sequence and a transglutaminase acceptor peptide sequence.

6. The process according to any one of the preceding claims, wherein said hyaluronan and/or said hyaluronan peptide conjugate and/or said second hyaluronan peptide conjugate are **characterized by** a mean molecular weight equal or greater than (≥) 250 kg/mol, ≥ 500 kg/mol, particularly greater than 1000 kg/mol (1 MDa); more particularly between 1000kg/mol and 4000 kg/mol, even more particularly between 1000 kg/mol and 2000 kg/mol.

7. A process for modification of a heparin or heparan sulfate polymer, wherein said heparin or heparan sulfate polymer comprises D-glucuronic acid moieties and L-iduronic acid moieties, each of which bearing reactive carboxylic acid moieties, and said process comprises the steps of:
a. thiolation of 5% to 20%, particularly 8-12%, more particularly approximately 10% of said reactive carboxylic acid moieties to yield partially thiolated heparin or heparan sulfate;
b. reacting said partially thiolated hyaluronan with divinylsulfone to yield vinylsulfone-heparin or vinylsulfone -heparan sulfate;
c. reacting said vinylsulfone-heparin or -heparan sulfate with a peptide comprising a cysteine moiety, wherein said peptide comprises a sequence selected from a transglutaminase donor peptide sequence and a transglutaminase acceptor peptide sequence.

8. The process according to any one of the preceding claims, wherein
a. the transglutaminase donor peptide sequence is or comprises a sequence selected from SEQ ID NO 01 to SEQ ID 14;
b. the transglutaminase acceptor peptide sequence is or comprises a sequence selected from SEQ ID NO 15 to SEQ ID 29.

9. A composition comprising or essentially consisting of a hyaluronan polymer of general formula I, 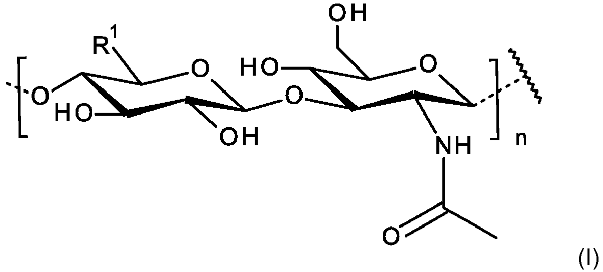 wherein 5% to 20%, particularly 8-12%, more particularly approximately 10% of R¹ moieties are represented by a general formula II, 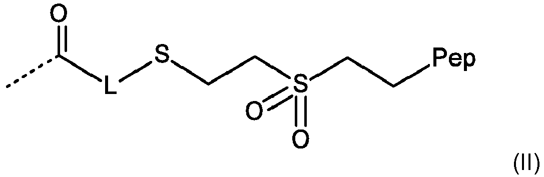 wherein
- L is a linker moiety, particularly a linker consisting of 2, 3, 4, 5 or 6 C, N and/or O atoms in the linking chain, more particularly L is NH-Alk or O-Alk or NH-NH-CO-Alk with Alk being a C1 to C5 alkyl, even more particularly L is -N-NHCO-(CH₂)₂-, and
- Pep is a transglutaminase donor peptide or a transglutaminase acceptor peptide, respectively, and the rest of R¹ moieties are represented by COOH.

10. A hyaluronan hydrogel comprising transglutaminase cross-linked hyaluronan and water, obtained
- by a process according to any one of claims 1 to 4, or
- by crosslinking a hyaluronan obtained by a process according to any one of claims 5 to 8, or
- by crosslinking a composition according to claims 9,
wherein the hydrogel comprises 0,25% to 0,99% of cross-linked hyaluronan in water (w/v), particularly 0,25% to 0,75% (w/v), or 0,4% to 0,6% (w/v), particularly ca. 0, 5% (w/v)
or
wherein the hydrogel comprises 0,5% to 4% of cross-linked hyaluronan in water (w/v), particularly 1% to 2% (w/v).

11. The hyaluronan hydrogel according to claims 10, **characterized by** a pore size of 0.1 µm to 1.0 µm, particularly 0.2 µm to 0.8 µm.

12. The hyaluronan hydrogel according to any one of claims 10 to 11, wherein said hydrogel is **characterized by** an adhesion strength, as measured by push-out assay,
c. of more than 0.5 kPa, in particular 1 to 10 kPa, more particularly around 2 kPa when adhesion to a cartilage surface is measured; and / or
d. of more than 4 kPa, particularly approx. 6 kPa when adhesion to a chondroitinase treated cartilage surface is measured.

13. The hyaluronan hydrogel according to any one of claims 10 to 12, **characterized by** a change in dimension (swelling or shrinking), as measured by comparing gel disk diameters after gelation and after additional swelling in an isotonic aqueous buffer for 3 days, of less than 20% on average, in particular less than 10% on average, more particularly less than 4%.

14. The hyaluronan hydrogel according to any one of claims 10 to 13, additionally comprising chondrogenic cells, particularly chondrocytes, chondroprogenitors, mesenchymal stem cells or adipose stem cells.

15. The hyaluronan hydrogel according to any one of claims 10 to 13, wherein said hydrogel comprises neurons, neural cells and/or neuroprogenitor cells.


<110>  ETH Zuerich

<120>  Transglutaminase Mediated High Molecular Weight Hyaluronan
       Hydrogels

<130>  eth147ep-1

<160>  39

<170>  PatentIn version 3.5

<210>  1
<211>  8
<212>  PRT
<213>  Homo sapiens


<220>
<221>  MOD_RES
<222>  (1)..(1)
<223>  ACETYLATION

<220>
<221>  MOD_RES
<222>  (1)..(1)
<223>  ACETYLATION

<220>
<221>  MOD_RES
<222>  (5)..(5)
<223>  AMIDATION

<400>  1

Phe Lys Gly Gly Glu Arg Cys Gly
1               5


<210>  2
<211>  5
<212>  PRT
<213>  Homo sapiens


<220>
<221>  MOD_RES
<222>  (1)..(1)
<223>  ACETYLATION

<220>
<221>  MOD_RES
<222>  (5)..(5)
<223>  AMIDATION

<400>  2

Phe Lys Gly Gly Cys
1               5


<210>  3

<211>  8
<212>  PRT
<213>  Homo sapiens

<220>
<221>  MOD_RES
<222>  (1)..(1)
<223>  ACETYLATION

<400>  3

Phe Lys Gly Gly Glu Arg Cys Gly
1               5

<210>  4
<211>  5
<212>  PRT
<213>  Homo sapiens

<220>
<221>  MOD_RES
<222>  (1)..(1)
<223>  ACETYLATION

<400>  4

Phe Lys Gly Gly Cys
1               5

<210>  5
<211>  5
<212>  PRT
<213>  Homo sapiens

<220>
<221>  MOD_RES
<222>  (1)..(1)
<223>  ACETYLATION

<400>  5

Leu Lys Gly Gly Cys
1               5

<210>  6
<211>  4
<212>  PRT
<213>  Homo sapiens

<220>
<221>  MISC_FEATURE
<222>  (1)..(1)
<223>  Dopa (L-3,4-dihydroxyphenylalanine)

<220>
<221>  MOD_RES

<222>  (1)..(1)
<223>  ACETYLATION

<400>  6

Xaa Lys Gly Cys
1


<210>  7
<211>  16
<212>  PRT
<213>  Homo sapiens


<220>
<221>  MOD_RES
<222>  (1)..(1)
<223>  ACETYLATION

<220>
<221>  MOD_RES
<222>  (16)..(16)
<223>  AMIDATION

<400>  7

Phe Lys Gly Gly Gly Pro Gln Gly Ile Trp Gly Gln Glu Arg Cys Gly
1               5                   10                  15


<210>  8
<211>  16
<212>  PRT
<213>  Homo sapiens


<220>
<221>  MOD_RES
<222>  (1)..(1)
<223>  ACETYLATION

<220>
<221>  MOD_RES
<222>  (16)..(16)
<223>  AMIDATION

<400>  8

Phe Lys Gly Gly Gly Pro Gln Gly Ile Ala Gly Gln Glu Arg Cys Gly
1               5                   10                  15


<210>  9
<211>  13
<212>  PRT
<213>  Homo sapiens


<220>
<221>  MOD_RES
<222>  (1)..(1)
<223>  ACETYLATION

<400>  9

Phe Lys Gly Gly Gly Pro Gln Gly Ile Trp Gly Gln Cys
1               5                   10


<210>  10
<211>  13
<212>  PRT
<213>  Homo sapiens


<220>
<221>  MOD_RES
<222>  (1)..(1)
<223>  ACETYLATION

<400>  10

Phe Lys Gly Gly Gly Pro Gln Gly Ile Ala Gly Gln Cys
1               5                   10


<210>  11
<211>  4
<212>  PRT
<213>  Homo sapiens


<220>
<221>  MOD_RES
<222>  (1)..(1)
<223>  ACETYLATION

<220>
<221>  MOD_RES
<222>  (4)..(4)
<223>  AMIDATION

<400>  11

Phe Lys Gly Cys
1


<210>  12
<211>  4
<212>  PRT
<213>  Homo sapiens


<220>
<221>  MOD_RES
<222>  (1)..(1)
<223>  ACETYLATION

<400>  12

Phe Lys Gly Cys
1

<210>  13
<211>  4
<212>  PRT
<213>  Homo sapiens

<220>
<221>  MOD_RES
<222>  (1)..(1)
<223>  ACETYLATION

<220>
<221>  MOD_RES
<222>  (4)..(4)
<223>  AMIDATION

<400>  13

Leu Lys Gly Cys
1

<210>  14
<211>  4
<212>  PRT
<213>  Homo sapiens

<220>
<221>  MOD_RES
<222>  (1)..(1)
<223>  ACETYLATION

<400>  14

Leu Lys Gly Cys
1

<210>  15
<211>  8
<212>  PRT
<213>  Homo sapiens

<400>  15

Asn Gln Glu Gln Val Ser Pro Leu
1               5

<210>  16
<211>  4
<212>  PRT
<213>  Homo sapiens

<400>  16

Gln Gln Leu Gly
1

<210>  17
<211>  11

<212>  PRT
<213>  Homo sapiens

<400>  17

Gly Gln Leu Lys His Leu Glu Gln Gln Glu Gly
1               5                   10


<210>  18
<211>  9
<212>  PRT
<213>  Homo sapiens

<400>  18

Asn Gln Glu Gln Val Ser Pro Leu Cys
1               5


<210>  19
<211>  12
<212>  PRT
<213>  Homo sapiens

<400>  19

Asn Gln Glu Gln Val Ser Pro Leu Glu Arg Cys Gly
1               5                   10


<210>  20
<211>  20
<212>  PRT
<213>  Homo sapiens

<400>  20

Asn Gln Glu Gln Val Ser Pro Leu Gly Pro Gln Gly Ile Trp Gly Gln
1               5                   10                  15


Glu Arg Cys Gly
            20


<210>  21
<211>  15
<212>  PRT
<213>  Homo sapiens

<400>  21

Asn Gln Glu Gln Val Ser Pro Leu Gly Gly Gly Glu Arg Cys Gly
1               5                   10                  15


<210>  22
<211>  12
<212>  PRT
<213>  Homo sapiens

<400>  22

Asn Gln Glu Gln Val Ser Pro Leu Asp Arg Cys Gly
1               5                   10

<210>  23
<211>  7
<212>  PRT
<213>  Homo sapiens

<220>
<221>  MOD_RES
<222>  (1)..(1)
<223>  ACETYLATION

<220>
<221>  MOD_RES
<222>  (5)..(5)
<223>  AMIDATION

<400>  23

Gly Gln Gln Gln Leu Gly Cys
1               5

<210>  24
<211>  10
<212>  PRT
<213>  Homo sapiens

<400>  24

Gly Gln Gln Gln Leu Gly Glu Arg Cys Gly
1               5                   10

<210>  25
<211>  10
<212>  PRT
<213>  Homo sapiens

<400>  25

Gly Gln Gln Gln Leu Gly Asp Arg Cys Gly
1               5                   10

<210>  26
<211>  7
<212>  PRT
<213>  Homo sapiens

<400>  26

Gly Gln Gln Gln Leu Gly Cys
1               5

<210>  27
<211>  12
<212>  PRT

<213>  Homo sapiens

<400>  27

Gly Gln Leu Lys His Leu Glu Gln Gln Glu Gly Cys
1               5                   10


<210>  28
<211>  15
<212>  PRT
<213>  Homo sapiens

<400>  28

Gly Gln Leu Lys His Leu Glu Gln Gln Glu Gly Glu Arg Cys Gly
1               5                   10                  15


<210>  29
<211>  15
<212>  PRT
<213>  Homo sapiens

<400>  29

Gly Gln Leu Lys His Leu Glu Gln Gln Glu Gly Asp Arg Cys Gly
1               5                   10                  15


<210>  30
<211>  19
<212>  DNA
<213>  Homo sapiens

<400>  30
aagtaccagg cagtgacag                                                    19


<210>  31
<211>  20
<212>  DNA
<213>  Homo sapiens

<400>  31
cctgtttccg tagcctcatg                                                   20


<210>  32
<211>  19
<212>  DNA
<213>  Homo sapiens

<400>  32
cagccgcttc acctacagc                                                    19


<210>  33
<211>  23
<212>  DNA
<213>  Homo sapiens

<400>  33

ttttgtattc aatcactgtc gcc                                                23

<210>  34
<211>  21
<212>  DNA
<213>  Homo sapiens

<400>  34
ggaattcggt gtggacatag g                                                  21

<210>  35
<211>  20
<212>  DNA
<213>  Homo sapiens

<400>  35
acttgggtcc tttgggtttg                                                    20

<210>  36
<211>  21
<212>  DNA
<213>  Homo sapiens

<400>  36
attcctagtg gctccaatgt g                                                  21

<210>  37
<211>  21
<212>  DNA
<213>  Homo sapiens

<400>  37
gcctacctcc atatgcattt t                                                  21

<210>  38
<211>  21
<212>  DNA
<213>  Homo sapiens

<400>  38
gaatgggaac cagcctatac c                                                  21

<210>  39
<211>  22
<212>  DNA
<213>  Homo sapiens

<400>  39
tctgtacttt cctctgttgc tg                                                 22